# EUROPÄISCHE PATENTSCHRIFT

(11) **EP 2 164 864 B1**
(45) Veröffentlichungstag und Bekanntmachung des Hinweises auf die Patenterteilung: **03.12.2014**
(21) Anmeldenummer: 08761043.2
(22) Anmeldetag: 16.06.2008
(51) Int. Cl.: C07K 14/435, C12N 15/12, A61K 8/64

(54) **SYNTHETISCHE REPETITIVE PROTEINE, DEREN HERSTELLUNG UND VERWENDUNG**
SYNTHETIC REPETITIVE PROTEINS, THE PRODUCTION AND USE THEREOF
PROTÉINES SYNTHÉTIQUES RÉPÉTITIVES, LEUR FABRICATION ET UTILISATION

(30) Priorität: 20.06.2007 EP 07110696
(43) Veröffentlichungstag der Anmeldung: 24.03.2010
(73) Patentinhaber: BASF SE, 67056 Ludwigshafen (DE)
(72) Erfinder: LIEBMANN, Burghard, 64625 Bensheim (DE); FEHR, Marcus, 67346 Speyer (DE); HÜMMERICH, Daniel, 67227 Frankenthal (DE)
(86) Internationale Anmeldenummer: PCT/EP2008/057526
(87) Internationale Veröffentlichungsnummer: WO 2008/155304

(56) Entgegenhaltungen:
- WO-A-2004/073644
- WO-A-2004/104021
- WO-A-2004/104042
- WO-A-2004/104043
- WO-A-2005/094868
- WO-A-2007/014755
- DATABASE UniProt [Online] 1. Juni 2001 (2001-06-01), "SubName: Full=Fibroin 3; Flags: Fragment;" XP002496497 gefunden im EBI accession no. UNIPROT:Q9BIT0 Database accession no. Q9BIT0
- ARDELL D H ET AL: "Tetntative identification of a resilin gene in Drosophila melanogaster" INSECT BIOCHEMISTRY AND MOLECULAR BIOLOGY, ELSEVIER SCIENCE LTD, GB, Bd. 31, Nr. 10, 1. September 2001 (2001-09-01), Seiten 965-970, XP002998361 ISSN: 0965-1748

### SEQUENCE LISTING

<110> BASF SE
<120> Synthetische repetitive Proteine, deren Herstellung und Verwendung
<130> PF59335
<160> 4
<170> PatentIn version 3.1
<210> 1
   <211> 1680
   <212> DNA
   <213> artificial sequence
<220>
   <223> Gen für repetitives R16 Protein
<220>
   <221> CDS
   <222> (1)..(1680)
   <223>
<400> 1
   atg gct agc atg act ggt gga cag caa atg ggt cgc gga tcc atg ggc      48
   Met Ala Ser Met Thr Gly Gly Gln Gln Met Gly Arg Gly Ser Met Gly
   1               5                   10                  15
   ccg ggt tct agc gcg gct gca gcc gcg gca gct gcg tcc ggc ccg ggt      96
   Pro Gly Ser Ser Ala Ala Ala Ala Ala Ala Ala Ala Ser Gly Pro Gly
               20                  25                  30
   cag ggc cag ggt cag ggt caa ggc cag ggt ggc cgt cct tct gac acc      144
   Gln Gly Gln Gly Gln Gly Gln Gly Gln Gly Gly Arg Pro Ser Asp Thr
           35                  40                  45
   tac ggc ccg ggt tct agc gcg gct gca gcc gcg gca gct gcg tcc ggc      192
   Tyr Gly Pro Gly Ser Ser Ala Ala Ala Ala Ala Ala Ala Ala Ser Gly
       50                  55                  60
   ccg ggt cag ggc cag ggt cag ggt caa ggc cag ggt ggc cgt cct tct      240
   Pro Gly Gln Gly Gln Gly Gln Gly Gln Gly Gln Gly Gly Arg Pro Ser
   65                  70                  75                  80
   gac acc tac ggc ccg ggt tct agc gcg gct gca gcc gcg gca gct gcg      288
   Asp Thr Tyr Gly Pro Gly Ser Ser Ala Ala Ala Ala Ala Ala Ala Ala
                   85                  90                  95
   tcc ggc ccg ggt cag ggc cag ggt cag ggt caa ggc cag ggt ggc cgt      336
   Ser Gly Pro Gly Gln Gly Gln Gly Gln Gly Gln Gly Gln Gly Gly Arg
               100                 105                 110
   cct tct gac acc tac ggc ccg ggt tct agc gcg gct gca gcc gcg gca      384
   Pro Ser Asp Thr Tyr Gly Pro Gly Ser Ser Ala Ala Ala Ala Ala Ala
           115                 120                 125
   gct gcg tcc ggc ccg ggt cag ggc cag ggt cag ggt caa ggc cag ggt      432
   Ala Ala Ser Gly Pro Gly Gln Gly Gln Gly Gln Gly Gln Gly Gln Gly
       130                 135                 140
   ggc cgt cct tct gac acc tac ggc ccg ggt tct agc gcg gct gca gcc      480
   Gly Arg Pro Ser Asp Thr Tyr Gly Pro Gly Ser Ser Ala Ala Ala Ala
   145                 150                 155                 160
   gcg gca gct gcg tcc ggc ccg ggt cag ggc cag ggt cag ggt caa ggc      528
   Ala Ala Ala Ala Ser Gly Pro Gly Gln Gly Gln Gly Gln Gly Gln Gly
                   165                 170                 175
   cag ggt ggc cgt cct tct gac acc tac ggc ccg ggt tct agc gcg gct      576
   Gln Gly Gly Arg Pro Ser Asp Thr Tyr Gly Pro Gly Ser Ser Ala Ala
               180                 185                 190
   gca gcc gcg gca gct gcg tcc ggc ccg ggt cag ggc cag ggt cag ggt      624
   Ala Ala Ala Ala Ala Ala Ser Gly Pro Gly Gln Gly Gln Gly Gln Gly
           195                 200                 205
   caa ggc cag ggt ggc cgt cct tct gac acc tac ggc ccg ggt tct agc      672
   Gln Gly Gln Gly Gly Arg Pro Ser Asp Thr Tyr Gly Pro Gly Ser Ser
       210                 215                 220
   gcg gct gca gcc gcg gca gct gcg tcc ggc ccg ggt cag ggc cag ggt      720
   Ala Ala Ala Ala Ala Ala Ala Ala Ser Gly Pro Gly Gln Gly Gln Gly
   225                 230                 235                 240
   cag ggt caa ggc cag ggt ggc cgt cct tct gac acc tac ggc ccg ggt      768
   Gln Gly Gln Gly Gln Gly Gly Arg Pro Ser Asp Thr Tyr Gly Pro Gly
                   245                 250                 255
   tct agc gcg gct gca gcc gcg gca gct gcg tcc ggc ccg ggt cag ggc      816
   Ser Ser Ala Ala Ala Ala Ala Ala Ala Ala Ser Gly Pro Gly Gln Gly
               260                 265                 270
   cag ggt cag ggt caa ggc cag ggt ggc cgt cct tct gac acc tac ggc      864
   Gln Gly Gln Gly Gln Gly Gln Gly Gly Arg Pro Ser Asp Thr Tyr Gly
           275                 280                 285
   ccg ggt tct agc gcg gct gca gcc gcg gca gct gcg tcc ggc ccg ggt      912
   Pro Gly Ser Ser Ala Ala Ala Ala Ala Ala Ala Ala Ser Gly Pro Gly
       290                 295                 300
   cag ggc cag ggt cag ggt caa ggc cag ggt ggc cgt cct tct gac acc      960
   Gln Gly Gln Gly Gln Gly Gln Gly Gln Gly Gly Arg Pro Ser Asp Thr
   305                 310                 315                 320
   tac ggc ccg ggt tct agc gcg gct gca gcc gcg gca gct gcg tcc ggc     1008
   Tyr Gly Pro Gly Ser Ser Ala Ala Ala Ala Ala Ala Ala Ala Ser Gly
                   325                 330                 335
   ccg ggt cag ggc cag ggt cag ggt caa ggc cag ggt ggc cgt cct tct     1056
   Pro Gly Gln Gly Gln Gly Gln Gly Gln Gly Gln Gly Gly Arg Pro Ser
               340                 345                 350
   gac acc tac ggc ccg ggt tct agc gcg gct gca gcc gcg gca gct gcg     1104
   Asp Thr Tyr Gly Pro Gly Ser Ser Ala Ala Ala Ala Ala Ala Ala Ala
           355                 360                 365
   tcc ggc ccg ggt cag ggc cag ggt cag ggt caa ggc cag ggt ggc cgt     1152
   Ser Gly Pro Gly Gln Gly Gln Gly Gln Gly Gln Gly Gln Gly Gly Arg
       370                 375                 380
   cct tct gac acc tac ggc ccg ggt tct agc gcg gct gca gcc gcg gca      1200
   Pro Ser Asp Thr Tyr Gly Pro Gly Ser Ser Ala Ala Ala Ala Ala Ala
   385                 390                 395                 400
   gct gcg tcc ggc ccg ggt cag ggc cag ggt cag ggt caa ggc cag ggt      1248
   Ala Ala Ser Gly Pro Gly Gln Gly Gln Gly Gln Gly Gln Gly Gln Gly
                   405                 410                 415
   ggc cgt cct tct gac acc tac ggc ccg ggt tct agc gcg gct gca gcc      1296
   Gly Arg Pro Ser Asp Thr Tyr Gly Pro Gly Ser Ser Ala Ala Ala Ala
               420                 425                 430
   gcg gca gct gcg tcc ggc ccg ggt cag ggc cag ggt cag ggt caa ggc      1344
   Ala Ala Ala Ala Ser Gly Pro Gly Gln Gly Gln Gly Gln Gly Gln Gly
           435                 440                 445
   cag ggt ggc cgt cct tct gac acc tac ggc ccg ggt tct agc gcg gct      1392
   Gln Gly Gly Arg Pro Ser Asp Thr Tyr Gly Pro Gly Ser Ser Ala Ala
       450                 455                 460
   gca gcc gcg gca gct gcg tcc ggc ccg ggt cag ggc cag ggt cag ggt      1440
   Ala Ala Ala Ala Ala Ala Ser Gly Pro Gly Gln Gly Gln Gly Gln Gly
   465                 470                 475                 480
   caa ggc cag ggt ggc cgt cct tct gac acc tac ggc ccg ggt tct agc      1488
   Gln Gly Gln Gly Gly Arg Pro Ser Asp Thr Tyr Gly Pro Gly Ser Ser
                   485                 490                 495
   gcg gct gca gcc gcg gca gct gcg tcc ggc ccg ggt cag ggc cag ggt      1536
   Ala Ala Ala Ala Ala Ala Ala Ala Ser Gly Pro Gly Gln Gly Gln Gly
               500                 505                 510
   cag ggt caa ggc cag ggt ggc cgt cct tct gac acc tac ggc ccg ggt      1584
   Gln Gly Gln Gly Gln Gly Gly Arg Pro Ser Asp Thr Tyr Gly Pro Gly
           515                 520                 525
   tct agc gcg gct gca gcc gcg gca gct gcg tcc ggc ccg ggt cag ggc      1632
   Ser Ser Ala Ala Ala Ala Ala Ala Ala Ala Ser Gly Pro Gly Gln Gly
       530                 535                 540
   cag ggt cag ggt caa ggc cag ggt ggc cgt cct tct gac acc tac ggc      1680
   Gln Gly Gln Gly Gln Gly Gln Gly Gly Arg Pro Ser Asp Thr Tyr Gly
   545                 550                 555                 560
<210> 2
   <211> 560
   <212> PRT
   <213> artificial sequence
<220>
   <223> Gen für repetitives R16 Protein
<400> 2
   Met Ala Ser Met Thr Gly Gly Gln Gln Met Gly Arg Gly Ser Met Gly
   1               5                   10                  15
   Pro Gly Ser Ser Ala Ala Ala Ala Ala Ala Ala Ala Ser Gly Pro Gly
               20                  25                  30
   Gln Gly Gln Gly Gln Gly Gln Gly Gln Gly Gly Arg Pro Ser Asp Thr
           35                  40                  45
   Tyr Gly Pro Gly Ser Ser Ala Ala Ala Ala Ala Ala Ala Ala Ser Gly
       50                  55                  60
   Pro Gly Gln Gly Gln Gly Gln Gly Gln Gly Gln Gly Gly Arg Pro Ser
   65                  70                  75                  80
   Asp Thr Tyr Gly Pro Gly Ser Ser Ala Ala Ala Ala Ala Ala Ala Ala
                   85                  90                  95
   Ser Gly Pro Gly Gln Gly Gln Gly Gln Gly Gln Gly Gln Gly Gly Arg
               100                 105                 110
   Pro Ser Asp Thr Tyr Gly Pro Gly Ser Ser Ala Ala Ala Ala Ala Ala
           115                 120                 125
   Ala Ala Ser Gly Pro Gly Gln Gly Gln Gly Gln Gly Gln Gly Gln Gly
       130                 135                 140
   Gly Arg Pro Ser Asp Thr Tyr Gly Pro Gly Ser Ser Ala Ala Ala Ala
   145                 150                 155                 160
   Ala Ala Ala Ala Ser Gly Pro Gly Gln Gly Gln Gly Gln Gly Gln Gly
                   165                 170                 175
   Gln Gly Gly Arg Pro Ser Asp Thr Tyr Gly Pro Gly Ser Ser Ala Ala
               180                 185                 190
   Ala Ala Ala Ala Ala Ala Ser Gly Pro Gly Gln Gly Gln Gly Gln Gly
           195                 200                 205
   Gln Gly Gln Gly Gly Arg Pro Ser Asp Thr Tyr Gly Pro Gly Ser Ser
       210                 215                 220
   Ala Ala Ala Ala Ala Ala Ala Ala Ser Gly Pro Gly Gln Gly Gln Gly
   225                 230                 235                 240
   Gln Gly Gln Gly Gln Gly Gly Arg Pro Ser Asp Thr Tyr Gly Pro Gly
                   245                 250                 255
   Ser Ser Ala Ala Ala Ala Ala Ala Ala Ala Ser Gly Pro Gly Gln Gly
               260                 265                 270
   Gln Gly Gln Gly Gln Gly Gln Gly Gly Arg Pro Ser Asp Thr Tyr Gly
   275 280 285
   Pro Gly Ser Ser Ala Ala Ala Ala Ala Ala Ala Ala Ser Gly Pro Gly
   290 295 300
   Gln Gly Gln Gly Gln Gly Gln Gly Gln Gly Gly Arg Pro Ser Asp Thr
   305 310 315 320
   Tyr Gly Pro Gly Ser Ser Ala Ala Ala Ala Ala Ala Ala Ala Ser Gly
   325 330 335
   Pro Gly Gln Gly Gln Gly Gln Gly Gln Gly Gln Gly Gly Arg Pro Ser
   340 345 350
   Asp Thr Tyr Gly Pro Gly Ser Ser Ala Ala Ala Ala Ala Ala Ala Ala
   355 360 365
   Ser Gly Pro Gly Gln Gly Gln Gly Gln Gly Gln Gly Gln Gly Gly Arg
   370 375 380
   Pro Ser Asp Thr Tyr Gly Pro Gly Ser Ser Ala Ala Ala Ala Ala Ala
   385 390 395 400
   Ala Ala Ser Gly Pro Gly Gln Gly Gln Gly Gln Gly Gln Gly Gln Gly
   405 410 415
   Gly Arg Pro Ser Asp Thr Tyr Gly Pro Gly Ser Ser Ala Ala Ala Ala
   420 425 430
   Ala Ala Ala Ala Ser Gly Pro Gly Gln Gly Gln Gly Gln Gly Gln Gly
   435 440 445
   Gln Gly Gly Arg Pro Ser Asp Thr Tyr Gly Pro Gly Ser Ser Ala Ala
   450 455 460
   Ala Ala Ala Ala Ala Ala Ser Gly Pro Gly Gln Gly Gln Gly Gln Gly
   465 470 475 480
   Gln Gly Gln Gly Gly Arg Pro Ser Asp Thr Tyr Gly Pro Gly Ser Ser
   485 490 495
   Ala Ala Ala Ala Ala Ala Ala Ala Ser Gly Pro Gly Gln Gly Gln Gly
   500 505 510
   Gln Gly Gln Gly Gln Gly Gly Arg Pro Ser Asp Thr Tyr Gly Pro Gly
           515                 520                 525
   Ser Ser Ala Ala Ala Ala Ala Ala Ala Ala Ser Gly Pro Gly Gln Gly
       530                 535                 540
   Gln Gly Gln Gly Gln Gly Gln Gly Gly Arg Pro Ser Asp Thr Tyr Gly
   545                 550                 555                 560
<210> 3
   <211> 1920
   <212> DNA
   <213> Artificial Sequence
<220>
   <223> Gen für repetitives S16 protein
<220>
   <221> CDS
   <222> (1)..(1920)
   <223>
<400> 3
   atg gct agc atg act ggt gga cag caa atg ggt cgc gga tcc atg ggt       48
   Met Ala Ser Met Thr Gly Gly Gln Gln Met Gly Arg Gly Ser Met Gly
   1               5                   10                  15
   tct gcg gct gca gcc gcg gca gct gcg ggt ccg ggc ggt ggc aac ggt       96
   Ser Ala Ala Ala Ala Ala Ala Ala Ala Gly Pro Gly Gly Gly Asn Gly
               20                  25                  30
   ggc cgt ccg tct gac acc tac ggt gcg ccg ggt ggc ggt aac ggt ggc      144
   Gly Arg Pro Ser Asp Thr Tyr Gly Ala Pro Gly Gly Gly Asn Gly Gly
           35                  40                  45
   cgt cct tct tcc tct tac ggt tct gcg gct gca gcc gcg gca gct gcg      192
   Arg Pro Ser Ser Ser Tyr Gly Ser Ala Ala Ala Ala Ala Ala Ala Ala
       50                  55                  60
   ggt ccg ggc ggt ggc aac ggt ggc cgt ccg tct gac acc tac ggt gcg      240
   Gly Pro Gly Gly Gly Asn Gly Gly Arg Pro Ser Asp Thr Tyr Gly Ala
   65                  70                  75                  80
   ccg ggt ggc ggt aac ggt ggc cgt cct tct tcc tct tac ggt tct gcg      288
   Pro Gly Gly Gly Asn Gly Gly Arg Pro Ser Ser Ser Tyr Gly Ser Ala
                   85                  90                  95
   gct gca gcc gcg gca gct gcg ggt ccg ggc ggt ggc aac ggt ggc cgt      336
   Ala Ala Ala Ala Ala Ala Ala Gly Pro Gly Gly Gly Asn Gly Gly Arg
               100                 105                 110
   ccg tct gac acc tac ggt gcg ccg ggt ggc ggt aac ggt ggc cgt cct      384
   Pro Ser Asp Thr Tyr Gly Ala Pro Gly Gly Gly Asn Gly Gly Arg Pro
           115                 120                 125
   tct tcc tct tac ggt tct gcg gct gca gcc gcg gca gct gcg ggt ccg      432
   Ser Ser Ser Tyr Gly Ser Ala Ala Ala Ala Ala Ala Ala Ala Gly Pro
       130                 135                 140
   ggc ggt ggc aac ggt ggc cgt ccg tct gac acc tac ggt gcg ccg ggt      480
   Gly Gly Gly Asn Gly Gly Arg Pro Ser Asp Thr Tyr Gly Ala Pro Gly
   145                 150                 155                 160
   ggc ggt aac ggt ggc cgt cct tct tcc tct tac ggt tct gcg gct gca      528
   Gly Gly Asn Gly Gly Arg Pro Ser Ser Ser Tyr Gly Ser Ala Ala Ala
                   165                 170                 175
   gcc gcg gca gct gcg ggt ccg ggc ggt ggc aac ggt ggc cgt ccg tct      576
   Ala Ala Ala Ala Ala Gly Pro Gly Gly Gly Asn Gly Gly Arg Pro Ser
               180                 185                 190
   gac acc tac ggt gcg ccg ggt ggc ggt aac ggt ggc cgt cct tct tcc      624
   Asp Thr Tyr Gly Ala Pro Gly Gly Gly Asn Gly Gly Arg Pro Ser Ser
           195                 200                 205
   tct tac ggt tct gcg gct gca gcc gcg gca gct gcg ggt ccg ggc ggt      672
   Ser Tyr Gly Ser Ala Ala Ala Ala Ala Ala Ala Ala Gly Pro Gly Gly
       210                 215                 220
   ggc aac ggt ggc cgt ccg tct gac acc tac ggt gcg ccg ggt ggc ggt      720
   Gly Asn Gly Gly Arg Pro Ser Asp Thr Tyr Gly Ala Pro Gly Gly Gly
   225                 230                 235                 240
   aac ggt ggc cgt cct tct tcc tct tac ggt tct gcg gct gca gcc gcg      768
   Asn Gly Gly Arg Pro Ser Ser Ser Tyr Gly Ser Ala Ala Ala Ala Ala
                   245                 250                 255
   gca gct gcg ggt ccg ggc ggt ggc aac ggt ggc cgt ccg tct gac acc      816
   Ala Ala Ala Gly Pro Gly Gly Gly Asn Gly Gly Arg Pro Ser Asp Thr
               260                 265                 270
   tac ggt gcg ccg ggt ggc ggt aac ggt ggc cgt cct tct tcc tct tac      864
   Tyr Gly Ala Pro Gly Gly Gly Asn Gly Gly Arg Pro Ser Ser Ser Tyr
           275                 280                 285
   ggt tct gcg gct gca gcc gcg gca gct gcg ggt ccg ggc ggt ggc aac      912
   Gly Ser Ala Ala Ala Ala Ala Ala Ala Ala Gly Pro Gly Gly Gly Asn
       290                 295                 300
   ggt ggc cgt ccg tct gac acc tac ggt gcg ccg ggt ggc ggt aac ggt      960
   Gly Gly Arg Pro Ser Asp Thr Tyr Gly Ala Pro Gly Gly Gly Asn Gly
   305                 310                 315                 320
   ggc cgt cct tct tcc tct tac ggt tct gcg gct gca gcc gcg gca gct     1008
   Gly Arg Pro Ser Ser Ser Tyr Gly Ser Ala Ala Ala Ala Ala Ala Ala
                   325                 330                 335
   gcg ggt ccg ggc ggt ggc aac ggt ggc cgt ccg tct gac acc tac ggt     1056
   Ala Gly Pro Gly Gly Gly Asn Gly Gly Arg Pro Ser Asp Thr Tyr Gly
               340                 345                 350
   gcg ccg ggt ggc ggt aac ggt ggc cgt cct tct tcc tct tac ggt tct     1104
   Ala Pro Gly Gly Gly Asn Gly Gly Arg Pro Ser Ser Ser Tyr Gly Ser
           355                 360                 365
   gcg gct gca gcc gcg gca gct gcg ggt ccg ggc ggt ggc aac ggt ggc     1152
   Ala Ala Ala Ala Ala Ala Ala Ala Gly Pro Gly Gly Gly Asn Gly Gly
       370                 375                 380
   cgt ccg tct gac acc tac ggt gcg ccg ggt ggc ggt aac ggt ggc cgt      1200
   Arg Pro Ser Asp Thr Tyr Gly Ala Pro Gly Gly Gly Asn Gly Gly Arg
   385                 390                 395                 400
   cct tct tcc tct tac ggt tct gcg gct gca gcc gcg gca gct gcg ggt      1248
   Pro Ser Ser Ser Tyr Gly Ser Ala Ala Ala Ala Ala Ala Ala Ala Gly
                   405                 410                 415
   ccg ggc ggt ggc aac ggt ggc cgt ccg tct gac acc tac ggt gcg ccg      1296
   Pro Gly Gly Gly Asn Gly Gly Arg Pro Ser Asp Thr Tyr Gly Ala Pro
               420                 425                 430
   ggt ggc ggt aac ggt ggc cgt cct tct tcc tct tac ggt tct gcg gct      1344
   Gly Gly Gly Asn Gly Gly Arg Pro Ser Ser Ser Tyr Gly Ser Ala Ala
           435                 440                 445
   gca gcc gcg gca gct gcg ggt ccg ggc ggt ggc aac ggt ggc cgt ccg      1392
   Ala Ala Ala Ala Ala Ala Gly Pro Gly Gly Gly Asn Gly Gly Arg Pro
       450                 455                 460
   tct gac acc tac ggt gcg ccg ggt ggc ggt aac ggt ggc cgt cct tct      1440
   Ser Asp Thr Tyr Gly Ala Pro Gly Gly Gly Asn Gly Gly Arg Pro Ser
   465                 470                 475                 480
   tcc tct tac ggt tct gcg gct gca gcc gcg gca gct gcg ggt ccg ggc      1488
   Ser Ser Tyr Gly Ser Ala Ala Ala Ala Ala Ala Ala Ala Gly Pro Gly
                   485                 490                 495
   ggt ggc aac ggt ggc cgt ccg tct gac acc tac ggt gcg ccg ggt ggc      1536
   Gly Gly Asn Gly Gly Arg Pro Ser Asp Thr Tyr Gly Ala Pro Gly Gly
               500                 505                 510
   ggt aac ggt ggc cgt cct tct tcc tct tac ggt tct gcg gct gca gcc      1584
   Gly Asn Gly Gly Arg Pro Ser Ser Ser Tyr Gly Ser Ala Ala Ala Ala
           515                 520                 525
   gcg gca gct gcg ggt ccg ggc ggt ggc aac ggt ggc cgt ccg tct gac      1632
   Ala Ala Ala Ala Gly Pro Gly Gly Gly Asn Gly Gly Arg Pro Ser Asp
       530                 535                 540
   acc tac ggt gcg ccg ggt ggc ggt aac ggt ggc cgt cct tct tcc tct      1680
   Thr Tyr Gly Ala Pro Gly Gly Gly Asn Gly Gly Arg Pro Ser Ser Ser
   545                 550                 555                 560
   tac ggt tct gcg gct gca gcc gcg gca gct gcg ggt ccg ggc ggt ggc      1728
   Tyr Gly Ser Ala Ala Ala Ala Ala Ala Ala Ala Gly Pro Gly Gly Gly
                   565                 570                 575
   aac ggt ggc cgt ccg tct gac acc tac ggt gcg ccg ggt ggc ggt aac      1776
   Asn Gly Gly Arg Pro Ser Asp Thr Tyr Gly Ala Pro Gly Gly Gly Asn
               580                 585                 590
   ggt ggc cgt cct tct tcc tct tac ggt tct gcg gct gca gcc gcg gca      1824
   Gly Gly Arg Pro Ser Ser Ser Tyr Gly Ser Ala Ala Ala Ala Ala Ala
           595                 600                 605
   gct gcg ggt ccg ggc ggt ggc aac ggt ggc cgt ccg tct gac acc tac      1872
   Ala Ala Gly Pro Gly Gly Gly Asn Gly Gly Arg Pro Ser Asp Thr Tyr
       610                 615                 620
   ggt gcg ccg ggt ggc ggt aac ggt ggc cgt cct tct tcc tct tac ggc      1920
   Gly Ala Pro Gly Gly Gly Asn Gly Gly Arg Pro Ser Ser Ser Tyr Gly
   625                 630                 635                 640
<210> 4
   <211> 640
   <212> PRT
   <213> Artificial Sequence
<220>
   <223> Gen für repetitives S16 protein
<400> 4
   Met Ala Ser Met Thr Gly Gly Gln Gln Met Gly Arg Gly Ser Met Gly
   1               5                   10                  15
   Ser Ala Ala Ala Ala Ala Ala Ala Ala Gly Pro Gly Gly Gly Asn Gly
               20                  25                  30
   Gly Arg Pro Ser Asp Thr Tyr Gly Ala Pro Gly Gly Gly Asn Gly Gly
           35                  40                  45
   Arg Pro Ser Ser Ser Tyr Gly Ser Ala Ala Ala Ala Ala Ala Ala Ala
       50                  55                  60
   Gly Pro Gly Gly Gly Asn Gly Gly Arg Pro Ser Asp Thr Tyr Gly Ala
   65                  70                  75                  80
   Pro Gly Gly Gly Asn Gly Gly Arg Pro Ser Ser Ser Tyr Gly Ser Ala
                   85                  90                  95
   Ala Ala Ala Ala Ala Ala Ala Gly Pro Gly Gly Gly Asn Gly Gly Arg
               100                 105                 110
   Pro Ser Asp Thr Tyr Gly Ala Pro Gly Gly Gly Asn Gly Gly Arg Pro
           115                 120                 125
   Ser Ser Ser Tyr Gly Ser Ala Ala Ala Ala Ala Ala Ala Ala Gly Pro
       130                 135                 140
   Gly Gly Gly Asn Gly Gly Arg Pro Ser Asp Thr Tyr Gly Ala Pro Gly
   145                 150                 155                 160
   Gly Gly Asn Gly Gly Arg Pro Ser Ser Ser Tyr Gly Ser Ala Ala Ala
                   165                 170                 175
   Ala Ala Ala Ala Ala Gly Pro Gly Gly Gly Asn Gly Gly Arg Pro Ser
   180 185 190
   Asp Thr Tyr Gly Ala Pro Gly Gly Gly Asn Gly Gly Arg Pro Ser Ser
   195 200 205
   Ser Tyr Gly Ser Ala Ala Ala Ala Ala Ala Ala Ala Gly Pro Gly Gly
   210 215 220
   Gly Asn Gly Gly Arg Pro Ser Asp Thr Tyr Gly Ala Pro Gly Gly Gly
   225 230 235 240
   Asn Gly Gly Arg Pro Ser Ser Ser Tyr Gly Ser Ala Ala Ala Ala Ala
   245 250 255
   Ala Ala Ala Gly Pro Gly Gly Gly Asn Gly Gly Arg Pro Ser Asp Thr
   260 265 270
   Tyr Gly Ala Pro Gly Gly Gly Asn Gly Gly Arg Pro Ser Ser Ser Tyr
   275 280 285
   Gly Ser Ala Ala Ala Ala Ala Ala Ala Ala Gly Pro Gly Gly Gly Asn
   290 295 300
   Gly Gly Arg Pro Ser Asp Thr Tyr Gly Ala Pro Gly Gly Gly Asn Gly
   305 310 315 320
   Gly Arg Pro Ser Ser Ser Tyr Gly Ser Ala Ala Ala Ala Ala Ala Ala
   325 330 335
   Ala Gly Pro Gly Gly Gly Asn Gly Gly Arg Pro Ser Asp Thr Tyr Gly
   340 345 350
   Ala Pro Gly Gly Gly Asn Gly Gly Arg Pro Ser Ser Ser Tyr Gly Ser
   355 360 365
   Ala Ala Ala Ala Ala Ala Ala Ala Gly Pro Gly Gly Gly Asn Gly Gly
   370 375 380
   Arg Pro Ser Asp Thr Tyr Gly Ala Pro Gly Gly Gly Asn Gly Gly Arg
   385 390 395 400
   Pro Ser Ser Ser Tyr Gly Ser Ala Ala Ala Ala Ala Ala Ala Ala Gly
   405 410 415
   Pro Gly Gly Gly Asn Gly Gly Arg Pro Ser Asp Thr Tyr Gly Ala Pro
   420 425 430
   Gly Gly Gly Asn Gly Gly Arg Pro Ser Ser Ser Tyr Gly Ser Ala Ala
           435                 440                 445
   Ala Ala Ala Ala Ala Ala Gly Pro Gly Gly Gly Asn Gly Gly Arg Pro
       450                 455                 460
   Ser Asp Thr Tyr Gly Ala Pro Gly Gly Gly Asn Gly Gly Arg Pro Ser
   465                 470                 475                 480
   Ser Ser Tyr Gly Ser Ala Ala Ala Ala Ala Ala Ala Ala Gly Pro Gly
                   485                 490                 495
   Gly Gly Asn Gly Gly Arg Pro Ser Asp Thr Tyr Gly Ala Pro Gly Gly
               500                 505                 510
   Gly Asn Gly Gly Arg Pro Ser Ser Ser Tyr Gly Ser Ala Ala Ala Ala
           515                 520                 525
   Ala Ala Ala Ala Gly Pro Gly Gly Gly Asn Gly Gly Arg Pro Ser Asp
       530                 535                 540
   Thr Tyr Gly Ala Pro Gly Gly Gly Asn Gly Gly Arg Pro Ser Ser Ser
   545                 550                 555                 560
   Tyr Gly Ser Ala Ala Ala Ala Ala Ala Ala Ala Gly Pro Gly Gly Gly
                   565                 570                 575
   Asn Gly Gly Arg Pro Ser Asp Thr Tyr Gly Ala Pro Gly Gly Gly Asn
               580                 585                 590
   Gly Gly Arg Pro Ser Ser Ser Tyr Gly Ser Ala Ala Ala Ala Ala Ala
           595                 600                 605
   Ala Ala Gly Pro Gly Gly Gly Asn Gly Gly Arg Pro Ser Asp Thr Tyr
       610                 615                 620
   Gly Ala Pro Gly Gly Gly Asn Gly Gly Arg Pro Ser Ser Ser Tyr Gly
   625                 630                 635                 640

## Beschreibung

Die vorliegende Erfindung betrifft neue synthetische, repetitive Proteine, sowie deren Herstellung und Verwendung.

### Stand der Technik

WO 2004/104043 beschreibt synthetische Bioelastomere, die von der repetitiven Sequenz von Resilin abgeleitet sind, sowie deren Verwendung. Ebenfalls beschrieben werden Hybridmoleküle, die aus Resilinsequenzen, sowie aus Sequenzen aus anderen Proteinen wie z.B. Spinnenseide zusammengesetzt sind.

WO2007/014755 beschreibt repetitive Proteine, welche Wiederholungseinheiten enthalten, die von Spinnenseideproteinen abgeleitet sind.

Die Eigenschaften von unterschiedlichen repetitiven Proteinen sind in der Literatur beschrieben. Beispielsweise sind Resiline für ihre guten elastischen Eigenschaften und den hohen Anteil elastisch gespeicherter Energie (resilience) bekannt (Gosline et al., Philosophical Transactions of the Royal Society of London - Series B: Biological Science, 357(1418): 121:32, 2002). Spinnenseiden sind unter anderem für ihre hohe Zugfestigkeit und Zähigkeit bekannt (Gosline et al.; J Exp Biol.; 202(Pt 23): 3295-303 (1999)).

### Beschreibung der Erfindung

Beschrieben wird ein repetitives Protein mit Wiederholungseinheiten, die die Konsensussequenz (I)
X₁ X₂ X₃ X₄ S X₅ X₆ Y G
wobei
X₁ G, Y, A oder N
X₂ G, L, Q
X₃ R, K, T oder P
X₄ P, A, T oder S
X₅ D, T oder S
X₆ S, Q oder T, ist und
die Konsensussequenz (II)
Z₁ Z₂ (Z₃A)ₙZ₄ Z₅ Z₆
wobei
Z₁ S, Q, N, T oder G
Z₂ keine Aminosäure oder A
Z₃ A oder G
Z₄ keine Aminosäure, A oder S
Z₅ G, S, Q, N oder T
Z₆ G, P, S, Q, N oder T
n eine natürliche ganze Zahl mit 2 ≤ n ≤ 12
ist, enthalten.

Repetitive Proteine sind dadurch gekennzeichnet, daß mindestens 60%, bevorzugt mindestens 80% ihrer Aminosäuresequenz aus Wiederholungseinheiten bestehen.

Eine Wiederholungseinheit ist eine Aminosäuresequenz mit einer Länge von 7-100, bevorzugt 12 - 60 und besonders bevorzugt 15 - 40 Aminosäuren, die innerhalb eines Proteins mehrere Mal als identische Sequenz oder als Variation mit 70% bevorzugt mindestens 80% und besonders bevorzugt mindestens 90% Identität vorkommt. Repetitive Proteine können identische Kopien oder Variationen einer einzigen oder mehrerer unterschiedlicher Aminosäuresequenzen enthalten.

Die Wiederholungseinheiten können durch Linker verbunden sein, die bevorzugt 1 bis 30 Aminosäuren, besonders bevorzugt 1 bis 20 Aminosäuren enthalten. Die Aminosäuresequenz eines Linkers kann von anderen Proteinen, bevorzugt Strukturproteinen abgeleitet sein oder kein natürliches Vorbild besitzen oder ganz abwesend sein.

Es können eine beliebige Anzahl von Wiederholungseinheiten, bevorzugt 1 - 100, besonders bevorzugt, 10 - 65 und am meisten bevorzugt 15 - 35 aneinandergefügt sein.

Der Begriff Konsensussequenz wie er hier verwendet wird, bezeichnet eine Aminosäuresequenz, die häufig an einer bestimmten Position vorkommende Aminosäuren enthält, wobei andere Aminosäuren nicht näher bestimmt werden sondern durch den Platzhalter X anstelle des üblich verwendeten Ein-Buchstaben-Codes für Aminosäuren ersetzt werden. Der Ein-Buchstaben-Code für Aminosäuren, der hier verwendet wird, ist dem Fachmann bekannt.

In einem Aspekt ist das Verhältnis der Anzahl der Konsensussequenzen (I) zur Anzahl der Konsensussequenzen (II) innerhalb 60%, bevorzugt mindestens 80% der Wiederholungseinheiten des repetitiven Proteins kleiner als fünf und größer als zwei.

In einem Aspekt ist das Verhältnis der Anzahl der Konsensussequenzen (I) zur Anzahl der Konsensussequenzen (II) innerhalb 60%, bevorzugt mindestens 80% der Wiederholungseinheiten des repetitiven Proteins gleich oder kleiner als zwei und größer als eins, bevorzugt gleich zwei.

In einem Aspekt ist das Verhältnis der Anzahl der Konsensussequenzen (I) zur Anzahl der Konsensussequenzen (II) innerhalb 60%, bevorzugt mindestens 80% der Wiederholungseinheiten des repetitiven Proteins gleich oder kleiner als eins, bevorzugt gleich eins.

In einem Aspekt enthalten 60%, bevorzugt mindestens 80% der Wiederholungseinheiten des repetitiven Proteins die Teilsequenz GGRPSDTYG oder GGRPSSSYG.

In einem Aspekt enthalten mindestens 60%, bevorzugt mindestens 80% der Wiederholungseinheiten der erfindungsgemäßen Proteine die Sequenzmotive An oder (GA)ₘ mit A = Alanin, G = Glycin, n = 2 - 12, m = 2-10, bevorzugt n = 5 - 10, m = 4 - 8 und besonders bevorzugt n = 7 - 9, m = 6-7.

Die repetitiven Proteine gemäß der vorliegenden Erfindung enthalten die Wiederholungseinheiten PGSSAAAAAAAASGPGQGQGQGQGQGGRPSDTYG oder SAAAAAAAAGPGGGNGGRPSDTYGAPGGGNGGRPSSSYG.

In einem weiteren Aspekt enthält das erfindungsgemäße repetitive Protein aminoterminal oder carboxyterminal eine Peptidsequenz von 4 - 30 und besonders bevorzugt 5 - 15 Aminosäuren Länge, die zur Detektion des Proteins mittels Immunoblotting oder zur Reinigung des Proteins über Affinitätschromatographie dient. Nichtlimitierende Beispiele für solche Peptidsequenzen sind 6 x His-Tag (HHHHHH), T7-Tag (MASMTGGQQMG), S-Tag (KETAAAKFERQHMDS), c-Myc-Tag (EQKLISEEDL), Strep-Tag (WSHPQFEK) oder HA-Tag (YPYDVPDYA) (Terpe; Appl Microbiol Biotechnol; 60(5): 523-33 (2003)). Zwischen dem erfindungsgemäßen Protein und der zusätzlichen Peptidsequenz können Aminosäuresequenzen eingefügt sein, die das chemische oder enzymatische Abspalten der Peptidsequenz ermöglichen.

In einer weiteren Ausführung enspricht die Aminosäuresequenz des repetitiven Proteins der SEQ ID NO 2 oder Teilen dieser Sequenz.

In einer weiteren Ausführung enspricht die Aminosäuresequenz des repetitiven Proteins der SEQ ID NO 4 oder Teilen dieser Sequenz.

Die erfindungsgemäßen repetitiven Proteine weisen überraschende neue Eigenschaften auf. Diese neuen Eigenschaften betreffen z.B. die Stabilität von wässrigen Proteinlösungen und die Assemblierungseigenschaften. Zudem wurde festgestellt, dass die neuen repetitiven Proteine kosmetischen oder dermatologischen Zusammensetzungen vorteilhafte Eigenschaften verleihen, insbesondere die hydratisierende oder weich machende Wirkung verbessern können. Die neuen repetitiven Proteine verhalten sich im übrigen wie gute Filmbildner und besitzen vor allem eine geringe Oberflächendichte.

Die vorliegende Erfindung betrifft daher auch die Verwendung von monomeren oder assemblierten neuen repetitiven Proteinen in der Kosmetik, in der Humanund Tierernährung für die Formulierung von Substanzen, für die Papier-, Lederund Textilveredelung und zum Beschichten von Oberflächen.

Die Herstellung von repetitiven Proteinen kann durch Expression natürlicher Gensequenzen erfolgen, die molekularbiologisch verändert wurden um die erfindungsgemäße Struktur zu erlangen. Methoden zur Isolierung und Modifikation natürlicher Gensequenzen sind dem Fachmann bekannt.

Bevorzugt erfolgt die Herstellung der repetitiven Proteine durch Expression synthetisch hergestellter Gensequenzen. Eine Möglichkeit zur Herstellung synthetischer Gensequenzen ist in Huemmerich et al., Biochemistry. 43(42):13604-12, (2004) beschrieben.

Ein weiterer Gegenstand der Erfindung betrifft Nukleinsäuresequenzen, die für die oben beschriebenen Proteine kodieren. Bevorzugte Nukleinsäuresequenzen sind SEQ ID NO 1 und SEQ ID NO 3.

Ein weiterer Gegenstand der Erfindung betrifft Expressionsvektoren, die die oben genannten Nukleinsäuresequenzen enthalten. Der Begriff Expressionsvektor bezeichnet im Allgemeinen ein genetisches Element, das in einen Wirtsorganismus eingebracht werden kann, und dort die Expression einer gewünschten Nukleotidsequenz ermöglicht. Beispiele für Expressionsvektoren sind Plasmide, Phagen oder Viren. Bevorzugt enthalten Expressionsvektoren regulative Elemente wie Promotoren oder Enhancer, die entsprechende kodierende Nukleotidsequenz, Transkriptionsinitiatoren und -terminatoren, sowie Elemente, die die Vervielfältigung des Vektors ermöglichen.

Die Expressionssysteme für Proteine sind wohlbekannt und wurden beschrieben in Sambrook et al.: Molecular cloning: A Laboratory Manual; 3rd Ed. Cold Spring Harbour Laboratory Press; Cold Spring Harbour (2001). Nichtlimitierende Beispiele für prokaryontische Expressionsorganismen sind Escherichia coli, Bacillus subtilis, Bacillus megaterium, Corynebacterium glutamicum u.a.. Nichtlimitierende Beispiele für eukaryontische Expressionsorganismen sind Hefen, wie Saccharomyces cerevisiae, Pichia pastoris u.a., filamentöse Pilze, wie Aspergillus niger, Aspergillus oryzae, Aspergillus nidulans, Trichoderma reesei, Acremonium chrysogenum u.a., Säugetierzellen, wie Hela-Zellen, COS-Zellen, CHO-Zellen u.a., Insektenzellen, wie Sf9-Zellen, MEL-Zellen u.a., Pflanzen oder Pflanzenzellen wie Solanum tuberosum, Nicotiana u.a..

Ein weiterer Gegenstand der Erfindung betrifft die Assemblierung der repetitiven Proteine zu Protein Microbeads, Nanofibrillen, Gelen und Filmen.

Protein-Microbeads lassen sich bevorzugt durch das im Folgenden beschriebene Verfahren herstellen:

Das repetitive Protein wird in einem ersten Lösungsmittel gelöst. Als Lösungsmittel können beispielsweise wässrige Salzlösungen verwendet werden. Insbesondere eignen sich hoch konzentrierte Salzlösungen mit einer Konzentration größer 2, insbesondere größer 4 und besonders bevorzugt größer 5 molar, deren Ionen stärker ausgeprägte chaotrope Eigenschaften aufweisen als Natrium- und Chloridionen. Ein Beispiel für eine solche Salzlösung ist 6 M Guanidiniumthiocyanat oder 9 M Lithiumbromid. Des Weiteren können organische Lösungsmittel zum Lösen der repetitiven Proteine verwendet werden. Insbesondere eignen sich fluorierte Alkohole oder zyklische Kohlenwasserstoffe. Beispiele dafür sind Hexafluorisopropanol und Cyclohexan. Die Herstellung der Protein-Microbeads kann in den beschriebenen Lösungsmitteln erfolgen. Alternativ kann dieses Lösungsmittel durch ein weiteres Lösungsmittel z.B. niedrig konzentrierte Salzlösungen (c < 0,5 M) durch Dialyse oder Verdünnung ersetzt werden. Die Endkonzentration des gelösten Proteins sollte zwischen 0,1 - 100 mg/ml betragen. Die Temperatur, bei der das Verfahren durchgeführt wird, beträgt üblicherweise 0 - 80, bevorzugt 5 - 50 und besonders bevorzugt 10 - 40 °C.

Bei Verwendung von wässrigen Lösungen können diese auch noch mit einem Puffer, bevorzugt im Bereich von pH 4 - 10, besonders bevorzugt 5 bis 9, ganz besonders bevorzugt 6 bis 8,5 versetzt sein.
Durch Zugabe eines Additivs wird eine Phasentrennung induziert. Dabei entsteht eine in der Mischung von Lösungsmittel und Additiv emulgierte proteinreiche Phase. Aufgrund von Oberflächeneffekten nehmen emulgierte proteinreiche Tröpfchen eine runde Form an. Durch die Wahl des Lösungsmittels, des Additivs und der Proteinkonzentration kann der mittlere Durchmesser der Protein-Microbeads auf Werte zwischen 0,1 µm bis 50 µm eingestellt werden.

Als Additiv können alle Substanzen verwendet werden, die einerseits mit dem ersten Lösungsmittel mischbar sind und andererseits die Bildung einer proteinreichen Phase induzieren. Wird die Microbeadbildung in organischen Lösungsmitteln durchgeführt, so eignen sich dafür organische Substanzen, die eine geringere Polarität als das Lösungsmittel aufweisen, z.B. Toluol. In wässrigen Lösungen können Salze als Additiv verwendet werden, deren Ionen stärker ausgeprägte kosmotrope Eigenschaften aufweisen als Natrium- und Chloridionen (z.B. Ammoniumsulfat; Kaliumphosphat). Die Endkonzentration des Additivs sollte abhängig von der Art des Additivs zwischen 1 % und 50 Gew.-% bezogen auf die Proteinlösung betragen.

Die proteinreichen Tröpfchen werden durch Aushärtung fixiert, wobei die runde Form erhalten bleibt. Die Fixierung beruht dabei auf der Ausbildung starker intermolekularer Wechselwirkungen. Die Art der Wechselwirkungen kann nichtkovalent, z.B. durch die Bildung intermolekularer β-Faltblattkristalle oder kovalent, z.B. durch chemische Quervernetzung sein. Die Aushärtung kann durch das Additiv und / oder durch die Zugabe einer weiteren geeigneten Substanz erfolgen. Die Aushärtung erfolgt bei Temperaturen zwischen 0 und 80°C, bevorzugt zwischen 5 und 60°C.

Diese weitere Substanz kann ein chemischer Quervernetzer sein. Unter einem chemischen Quervernetzer wird dabei ein Molekül verstanden, bei dem mindestens zwei chemisch reaktive Gruppen über einen Linker miteinander verbunden sind. Beispiele dafür sind Sulfhydryl-reaktive Gruppen (z.B. Maleimide, Pydridyldisulfide, α -Haloacetyle, Vinylsulfone, Sulfatoalkylsulfone (bevorzugt Sulfatoethylsulfone), Amin-reaktive Gruppen (z.B. Succinimidylester, Carbodiimde, Hydroxymethyl- Phosphin, Imidoester, PFP-Ester, Aldehyde, Isothiocyanate etc.), Carboxy-reaktive Gruppen (z.B. Amine etc.), Hydroxyl-reaktive Gruppen (z.B. Isocyanate etc.), unselektive Gruppen (z.B. Arylazide etc.) und photoaktivierbare Gruppen (z.B. Perfluorphenylazid etc.). Diese reaktiven Gruppen können mit in Proteinen vorhandenen Amin-, Thiol-, Carboxyl- oder Hydroxylgruppen kovalente Verknüpfungen bilden.

Zur Aushärtung der Microbeads können auch Substanzen verwendet werde, die eine kovalente Verknüpfung zwischen Aminosäureresten bilden. Beispiel dafür ist eine Kombination von Ammoniumperoxodisulfat und Tris(2,2'-bipyridyl)dichlororuthenium(II), die unter Einwirkung von sichtbarem Licht die Bildung von Dityrosinen vermitteln.

Die stabilisierten Microbeads werden mit einem geeigneten weiteren Lösungsmittel, z.B. Wasser gewaschen und anschließend durch dem Fachmann geläufige Verfahren getrocknet, z.B. durch Lyophilisierung oder Sprühtrocknung. Der Erfolg der Kugelbildung wird mit Hilfe der Rasterelektronenmikroskopie überprüft.

Das Verfahren zur Herstellung von Microbeads erlaubt das Einschließen und damit eine Formulierung von hydrophoben Wirkstoffen in die Microbeads. Dieser Prozess umfasst zwei Schritte. Im ersten Schritt werden der hydrophobe Wirkstoff und das repetitive Protein in einer gemeinsamen Phase gelöst. Dazu können der Wirkstoff und das Protein direkt durch ein Lösungsmittel oder eine Lösungsmittelmischung in Lösung gebracht werden. Alternativ können der Wirkstoff und das Protein zunächst in unterschiedlichen Lösungsmitteln gelöst und die Lösungen im Anschluss miteinander vermischt werden, so dass wiederum eine gemeinsame Phase entsteht. Bei der gemeinsamen Phase kann es sich um eine molekular-disperse Phase oder eine kolloidal-disperse Phase handeln.

Das Lösen des hydrophoben Wirkstoffes und des Proteins in verschiedenen Lösungsmitteln und das anschließende Mischen beider Lösungen ist insbesondere dann von Vorteil, wenn sich der hydrophobe Wirkstoff und das Protein nicht in einem gemeinsamen Lösungsmittel oder Lösungsmittelgemisch lösen lassen. Auf diese Art und Weise lassen sich durch dieses Vorgehen auch kolloidal-disperse Lösungen hydrophober Wirkstoffe herstellen, indem der in einem geeigneten Lösungsmittel gelöste Wirkstoff in ein anderes Lösungsmittel verdünnt wird, in dem dieser Wirkstoff unlöslich ist.

Da Proteine in der Regel gut wasserlöslich sind, wird bevorzugt mit wässrigen Lösungen und Mischungen aus Wasser und wassermischbaren, organischen Lösungsmitteln gearbeitet. Bespiele für geeignete, wassermischbare Lösungsmittel sind Alkohole wie Methanol, Ethanol und Isopropanol, fluorierte Alkohole wie Hexafluorisopropanol und Trifluorethanol, Alkanone wie Aceton oder auch Sulfoxide wie z.B. Dimethylsulfoxid oder Formamide wie Dimethylformamid oder andere organische Lösungsmittel wie z.B. Tetrahydrofuran und Acetonitril oder N-Methyl-2-pyrrolidon. Im allgemeinen kann mit allen Lösungsmitteln und Lösungsmittelgemischen gearbeitet werden, in denen sich die erfindungsgemäßen Proteine lösen lassen. Beispiele für geeignete Lösungsmittel sind fluorierte Alkohole wie z.B. Hexafluorisopropanol oder Trifluorethanol, ionische Flüssigkeiten wie z.B. EMIM-Acetat, wässrige Lösungen chaotroper Salze wie z.B. Harnstoff, Guanidiuniumhydrochlorid und Guanidiniumthiocyanat oder organische Säuren wie z.B. Ameisensäure sowie Mischungen dieser Lösungsmittel mit anderen organischen Lösungsmitteln. Beispiele für Lösungsmittel, die sich mit den Lösungsmitteln für das Protein mischen lassen sind u.a. Alkohole wie Methanol, Ethanol und Isopropanol, Alkanone wie Aceton, Sulfoxide wie z.B. Dimethylsulfoxid, Formamide wie Dimethylformamid, Halogenalkane wie Methylenchlorid oder auch weitere organische Lösungsmittel wie z.B. Tetrahydrofuran.

In einem zweiten Schritt wird in der gemeinsamen Wirkstoff - Protein Lösung eine Phasentrennung und die anschließende Aushärtung der Microbeads induziert. Dieser Prozess erfolgt nach dem gleichen Prinzip, wie bereits für die Herstellung von reinen Protein-Microbeads beschrieben wurde. Während der Microbeadbildung findet eine Wechselwirkung der hydrophoben Wirkstoffe mit den erfindungsgemäßen repetitiven Proteinen statt.

Die Wechselwirkungen zwischen dem hydrophoben Wirkstoff und dem Protein beruht im wesentlichen auf ihren hydrophoben Eigenschaften, wobei allerdings auch Wasserstoffbrücken, ionische Wechselwirkungen und van-der-Waals-Wechselwirkungen beteiligt sein können. Der hydrophobe Wirkstoff kann an die Oberfläche gebunden sein, in die Microbeads eingeschlossen sein oder auch auf beide Arten mit den Microbeads assoziiert sein. Die Bindung des hydrophoben Wirkstoffs an die Microbeads kann durch die Verarmung des Assemblierungsansatzes an gelöstem Wirkstoff bestimmt werden. Die Konzentration des Wirkstoffs kann durch eine quantitative Analyse seiner Eigenschaften gemessen werden. So kann die Bindung von lichtabsorbierenden Wirkstoffen z.B. durch photometrische Methoden analysiert werden. Dazu werden z.B. die Färbung der Microbeads oder die Entfärbung der protein- und wirkstoffarmen Phase des Formulierungsansatzes durch Messen der Absorption des farbigen Wirkstoffs bestimmt. Mit Hilfe dieser Methoden kann auch bestimmt werden, wie hoch der Wirkstoffanteil in den Microbeads ist.

Die Freisetzung der Wirkstoffe aus den Microbeads kann durch Desorption in geeignete Lösungsmittel, durch den Abbau der Microbeads durch Proteasen oder durch Auflösen der Microbeads durch geeignete Lösungsmittel erfolgen. Geeignete Lösungsmittel für die Desorption sind alle Lösungsmittel oder Lösungsmittelgemische, in denen sich der Wirkstoff lösen lässt. Geeignete Proteasen können als technische Proteasen einer Suspension von Protein-Microbeads gezielt zugesetzt werden oder am gewünschten Wirkort der Effektormoleküle natürlicherweise vorkommen, wie z.B. Hautproteasen, Proteasen des Verdauungstraktes, z.B. Magen- oder Darmproteasen oder von Mikroorganismen freigesetzte Proteasen. Lösungsmittel, die die Microbeads auflösen können, sind z.B. fluorierte Alkohole wie z.B. Hexafluorisopropanol oder Trifluorethanol, ionische Flüssigkeiten wie z.B. EMIM-Acetat, wässrige Lösungen chaotroper Salze wie z.B. Harnstoff, Guanidiuniumhydrochlorid und Guanidiniumthiocyanat oder organische Säuren wie z.B. Ameisensäure sowie Mischungen dieser Lösungsmittel mit anderen organischen Lösungsmitteln. Die Geschwindigkeit und die Kinetik der Freisetzung der Effektormoleküle können z.B. durch die Beladungsdichte mit Wirkstoffen und die Größe der Microbeads bzw. ihrem Verhältnis von Volumen zur Oberfläche gesteuert werden.

Unter geeigneten Bedingungen lassen sich aus den erfindungsgemäßen repetitiven Proteinen Nanofibrillen herstellen. Die Nanofibrillenbildung äußert sich auf makroskopischer Ebene in einer gelartigen Verdickung einer wässrigen Proteinlösung ab einer Nanofibrillenkonzentration von 1 mg/ml.

Ausgangspunkt für die Assemblierung von Proteinnanofibrillen ist eine wässrige Lösung des repetitiven Proteins mit einer Proteinkonzentration von 0,1 - 400 mg/ml, bevorzugt 1 - 100 mg/ml und besonders bevorzugt 5 - 20 mg/ml. Die wässrige Lösung kann mit einem Puffer, bevorzugt im Bereich von pH 4 - 10, besonders bevorzugt 5 bis 9, ganz besonders bevorzugt 6 bis 8,5 versetzt sein. Die Assemblierung erfolgt von selbst innerhalb von mehreren Wochen. Sie kann beschleunigt werden durch die Zugabe von wasserlöslichen organischen Lösungsmitteln, die die Polarität der Lösung herabsetzen. Beispiele dafür sind Alkohole wie Ethanol und Methanol. Die Endkonzentration des Lösungsmittels sollte zwischen 1% und 50 Gew.-% bezogen auf die Proteinlösung betragen.

Die assemblierten Proteinnanofibrillen werden mit einem geeigneten Lösungsmittel, z.B. Wasser gewaschen und anschließend durch dem Fachmann geläufige Verfahren getrocknet, z.B. durch Lyophilisierung, Kontakttrocknung oder Sprühtrocknung. Der Erfolg der Faserbildung wird mit Hilfe der Transmissionselektronenmikroskopie oder der Rasterkraftmikroskopie überprüft.

Unter geeigneten Bedingungen können Filme aus den erfindungsgemäßen repetitiven Proteinen hergestellt werden. Dazu wird das repetitive Protein in einem ersten Lösungsmittel gelöst. Als Lösungsmittel können beispielsweise wässrige Salzlösungen verwendet werden. Insbesondere eignen sich hoch konzentrierte Salzlösungen mit einer Konzentration größer 2, insbesondere größer 4 und besonders bevorzugt größer 5 molar, deren Ionen stärker ausgeprägte chaotrope Eigenschaften aufweisen als Natrium- und Chloridionen. Ein Beispiel für eine solche Salzlösung ist 6 M Guanidiniumthiocyanat oder 9 M Lithiumbromid. Des Weiteren können organische Lösungsmittel zum Lösen der repetitiven Proteine verwendet werden. Insbesondere eignen sich fluorierte Alkohole oder zyklische Kohlenwasserstoffe. Beispiele dafür sind Hexafluorisopropanol und Cyclohexan. Die Herstellung der Filme kann in dem beschriebenen ersten Lösungsmittel erfolgen, sofern dieses keine nichtflüchtigen Substanzen enthält. Lösungsmittel, die nichtflüchtige Bestandteile enthalten, müssen durch ein weiteres Lösungsmittel, das keine nichtflüchtigen Substanzen enthält z.B. durch Dialyse ersetzt werden. Bevorzugte flüchtige Lösungsmittel, aus denen die Filmbildung erfolgen kann sind Hexafluorisopropanol, Cyclohexan, Ameisensäure und Wasser. Die Endkonzentration des gelösten Proteins sollte zwischen 1 - 200 mg/ml, bevorzugt zwischen 20 - 150 mg/ml und besonders bevorzugt zwischen 50 - 100 mg/ml betragen. Die Temperatur, bei der das Verfahren durchgeführt wird, beträgt üblicherweise 0 - 80, bevorzugt 5 - 50 und besonders bevorzugt 10 - 40 °C.
Das in dem flüchtigen Lösungsmittel gelöste repetitive Protein wird auf einer glatten Oberfläche ausplattiert. Das Lösungsmittel verdunstet bei Raumtemperatur und läßt den Proteinfilm zurück.

Frisch hergestellte Proteinfilme sind in der Regel wasserlöslich. Zur Stabilisierung der Filme können diese mit Substanzen inkubiert werden, die die Ausbildung stabiler Sekundär-, Tertiär- und Quartärstrukturen innerhalb des Films induzieren. Beispiele für solche Substanzen sind Alkohole wie z.B. Methanol und Ethanol oder Lösungen von Salzen, deren Ionen stärker ausgeprägte kosmotrope Eigenschaften aufweisen als Natrium- und Chloridionen (z.B. Ammoniumsulfat; Kaliumphosphat). Nach der Stabilisierung der Filme können diese mit geeigneten Lösungsmitteln wie z.B. Alkoholen oder Wasser gewaschen und anschließend getrocknet werden.

Beschrieben sind auch Moleküle, die aus Kupplungen aus einem erfindungsgemäßen repetitiven Protein und einem Effektormolekül bestehen. Als Effektormoleküle werden Moleküle verstanden, die eine bestimmte, vorhersehbare Wirkung aufweisen. Dies können entweder proteinartige Moleküle sein, wie Enzyme oder auch nicht-proteinogene Moleküle wie Farbstoffe, Lichtschutzmittel, Vitamine, Provitamine, Antioxidantien und Fettsäuren, Conditioner oder Metallionen-enthaltende Verbindungen.

Die Effektormoleküle sind mit dem repetitiven Protein verbunden. Die Verbindung zwischen Effektormolekül und Protein und kann sowohl eine kovalente Bindung sein als auch auf ionischen oder van-der Waals Wechselwirkungen oder hydrophoben Wechselwirkungen oder Wasserstoffbrückenbindungen oder Adsorption beruhen.

Die kovalente Verknüpfung des Effektormoleküls kann über die Seitenketten der Polypeptidsequenz des repetitiven Proteins erfolgen, insbesondere über Aminofunktionen oder Hydroxyfunktionen oder Carboxylatfunktionen oder Thiolfunktionen. Bevorzugt ist eine Verknüpfung über die Aminofunktionen von einem oder mehreren Lysinresten, über die Carboxylatfunktionen von einem oder mehreren Glutamat- oder Aspartat-resten, über die Thiolfunktion von einem oder mehreren Cysteinresten oder über die N-terminale oder C-terminale Funktion des repetitiven Proteins. Außer den in der Aminosäuresequenz vorkommenden Aminosäurefunktionen können auch Aminosäuren mit geeigneten Funktionen (z.B. Cysteine, Lysine, Aspartate, Glutamate) an die Sequenz angefügt oder in die Sequenz eingefügt werden, oder Aminosäuren des repetitiven Proteins durch solche Aminosäurefunktionen substituiert werden.

Die Verknüpfung der Effektormoleküle mit dem repetitiven Protein kann direkt, d.h. als kovalente Verknüpfung zweier bereits vorhandener chemischer Funktionen erfolgen. Dabei werden die oben beschriebenen chemischen Funktionen der repetitiven Proteine mit reaktiven Gruppen verknüpft, die in den Effektormolekülen enthalten sind. Beispiele für solche reaktiven Gruppen sind Sulfhydryl-reaktive Gruppen, z.B. Maleimide, Pydridyldisulfide, α-Haloacetyle, Vinylsulfone, Sulfatoalkylsulfone (bevorzugt Sulfatoethylsulfone), Amin-reaktive Gruppen (z.B. Succinimidylester, Carbodiimde, Hydroxymethyl-Phosphin, Imidoester, PFP-Ester, Aldehyd, Isothiocyanat etc.), Carboxy-reaktive Gruppen (z.B. Amine etc.), Hydroxyl-reaktive Gruppen (z.B. Isocyanate etc.), unselektive Gruppen (z.B. Arylazide etc.) und photoaktivierbare Gruppen (z.B. Perfluorphenylazid etc.).

Besitzen die zu koppelnden Funktionen der erfindungsgemäßen repetitiven Proteine und Effektoren eine zu geringe Reaktivität für eine direkte Kopplung, so könnnen diese Funktionen mit dem Fachmann geläufigen Methoden aktiviert werden (z.B. Aktivierung von Carboxylfunktionen mit Carbodiimiden).

Die Verknüpfung kann aber auch über einen sogenannten Linker, d.h. einem mindestens bifunktionellen Molekül erfolgen, der mit einer oder mehreren Funktionen mit dem Effektormolekül eine Bindung eingeht und mit einer oder mehreren anderen Funktionen mit dem repetitiven Protein verknüpft wird. Die Verwendung solcher massgeschneiderter Linker erlaubt die genaue Anpassung der Verknüpfung an das gewünschte Effektormolekül. Ausserdem ist es dadurch möglich, mehrere Effektormoleküle mit einem repetitiven Protein definiert zu verknüpfen.

Der verwendete Linker richtet sich nach der zu koppelnden Funktionalität. Geeignet sind z.B. Moleküle, die an ein repetitives Protein koppeln mittels Sulfhydryl-reaktiven Gruppen, z.B. Maleimide, Pydridyldisulfide, α-Haloacetyle, Vinylsulfone, Sulfatoalkylsulfone (bevorzugt Sulfatoethylsulfone), Amin-reaktive Gruppen (z.B. Succinimidylester, Carbodiimde, Hydroxymethyl- Phosphin, Imidoester, PFP-Ester, Aldehyd, Isothiocyanat etc.), Carboxy-reaktive Gruppen (z.B. Amine etc.), Hydroxyl-reaktive Gruppen (z.B. Isocyanate etc.), unselektive Gruppen (z.B. Arylazide etc.) und photoaktivierbare Gruppen (z.B. Perfluorphenylazid etc.) und zu Effektormolekülen koppeln mittels
- Sulfhydryl-reaktiven Gruppen (z.B. Maleimide, Pydridyldisulfide, α-Haloacetyle, Vinylsulfone, Sulfatoalkylsulfone (bevorzugt Sulfatoethylsulfone)
- Amin-reaktive Gruppen (z.B. Succinimidylester, Carbodiimde, Hydroxymethyl- Phosphin, Imidoester, PFP-Ester, Aldehyd, Isothiocyanat etc.)
- Zucker bzw. oxidierte Zucker-reaktive Gruppen (z.B. Hydrazide etc.)
- Carboxy-reaktive Gruppen (z.B. Amine etc.)
- Hydroxyl-reaktive Gruppen (z.B. Isocyanate etc.)
- Thymin-reaktive Gruppen (z.B. Psoralen etc.)
- unselektive Gruppen (z.B. Arylazide etc.)
- photoaktivierbare Gruppen (z.B. Perfluorphenylazid etc.)
- Metallkomplexierenden Gruppen (z.B. EDTA, Hexahis, Ferritin)
- Antikörper und -fragmente (z.B. single-chain antibodies, F(ab)-Fragmente von Antikörpern, katalytische Antikörper).

Die chemischen Funktionen eines Linkers können durch Spacerelemente verbunden sein. Spacerelemente können beispielsweise aus Alkylketten, Ethylenglykol und Polyethylenglykolen zusammengesetzt sein.

Besonders bevorzugt sind Linker- und/oder Spacerelemente, die eine potentielle Spaltstelle für eine Protease, Lipase, Esterase, Phosphatase, Hydrolase besitzen, d.h. enzymatisch spaltbar sind.

Beispiele für enzymatisch spaltbare Linker, die bei den erfindungsgemässen Molekülen eingesetzt werden können, sind beispielsweise in WO 98/01406 genannt, auf deren gesamten Inhalt hiermit ausdrücklich Bezug genommen wird.

Besonders bevorzugt sind Linker und Spacer die thermospaltbar oder photospaltbar sind. Entsprechende chemische Strukturen sind dem Fachmann bekannt und werden zwischen die Molekülteile Effektormolekül und repetitives Protein integriert.

Falls das Effektormolekül aus einer Polypeptidsequenz besteht, kann die Verknüpfung von Effektor und repetitivem Protein als ein sogenanntes Fusionsprotein erfolgen, d.h. es wird eine durchgängige Polypeptidsequenz verwendet, die sich aus Effektor- und repetitiven Proteinteilsequenzen zusammensetzt.

Es können zwischen Effektor und repetitivem Protein auch noch sogenannte Spacerelemente eingebaut werden, beispielsweise Polypeptidsequenzen, die eine potentielle Spaltstelle für eine Protease, Lipase, Esterase, Phosphatase, Hydrolase besitzen oder Oligo- und Polypeptidsequenzen, die eine leichte Aufreinigung des Fusionsproteins gestatten, beispielsweise sogenannte Histags, d.h. Oligohistidinreste.

Die an repetitive Proteine kovalent oder nicht-kovalent gekoppelten Effektormoleküle können in ihrer gebundenen Form aktiv sein. Alternativ können die an repetitive Proteine gekoppelten Effektormoleküle aber auch von den repetitiven Proteinen freigesetzt werden.

Die Freisetzung kovalent gekoppelter Effektormoleküle von den repetitiven Proteinen kann durch Spaltung spezifisch eingebrachter spaltbarer Spacer oder Kopplungslinker, die z.B. thermospaltbar, photospaltbar oder enzymatisch spaltbar sein können, aber auch durch proteolytischen Abbau (z.B. durch Proteasen) erfolgen.

### Experimenteller Teil

### Beispiel 1

### Herstellung des repetitiven Proteins R₁₆ (SEQ ID NO:2)

Ein synthetisches Gen, das für das repetitive Protein R₁₆ kodiert, wurde durch Multimerisierung eines synthetischen Oligonukleotides R: entspreched der in Huemmerich et al.; Biochemistry 43(42):13604-12, (2004) beschriebenen Methode zu einem 16mer multimerisiert und in den Expressionsvektor pET21a (Novagen) kloniert. Die Expression erfolgte in dem Stamm E.coli BLR [DE3] (Novagen).
Die Anzucht und Proteinsynthese erfolgte bei pO₂>20% und pH=6,8 im Fed-Batch Verfahren.

### Medium

| 8 Liter | Wasser |
|---|---|
| 230 g | Hefe-Extrakt |
| 150 g | Bacto-Trypton |
| 30 g | Glycerin (99 %) |
| 20 g | Kaliumdihydrogenphosphat (KH₂PO₄) |
| 50 g | Ammoniumsulfat ((NH₄)₂SO₄) |
| 10,5 g | Magnesiumsulfatheptahydrat (MgSO₄ * 7 H₂O) |
| 1,0 g | Calciumchloriddihydrat (CaCl₂ * 2 H₂O) |
| 100 ml | Spurensalzlösung |
| | mit Wasser auf 9,7 Liter auffüllen pH-Wert mit 25%iger NaOH auf 6,8 einstellen |
| 3 ml | Tego KS 911 (Antifoam; Goldschmidt Produkte) |
| 1 g | Ampicillin |

### Spurensalzlösung:

| 5 Liter | Wasser |
|---|---|
| 200,00 g | Citronensäuremonohydrat |
| 55,00 g | ZnSO₄ * 7 H₂O |
| 42,50 g | (NH₄)₂Fe(SO₄)₂ * 6 H₂O |
| 15,00 g | MnSO₄*H₂O |
| 4,00 g | CuSO₄*5H₂O |
| 1,25 g | CoSO₄*7H₂O |

### Feeding Lösung

| | |
|---|---|
| 800 g | Wasser |
| 30 g | Citronensäure Monohydrat |
| 60 g | Natriumsulfat (Na₂SO₄) |
| 4,5 g | (NH₄)₂Fe(SO₄)₂ *6H₂O |
| 3400 g | Glycerin 99,5 % |

Nach Aufbrauchen des im Grundmedium enthaltenen Glycerins wurde ein konstanter Feed bei einer Rate von 100 ml/h gestartet.

Die Proteinsynthese wurde durch Zugabe von 1 mM Isopropyl-β-D-thiogalactopyranosid induziert, nachdem die Bakterienkultur eine optische Dichte OD₆₀₀=40 erreichte. Zu diesem Zeitpunkt wurde die Temperatur der Kultur von 37°C auf 32°C gesenkt. 7 h nach Induktion wurden die Zellen geerntet.

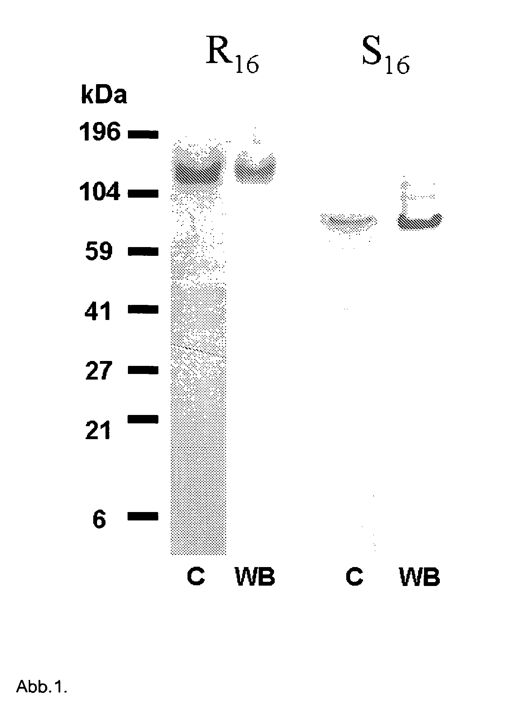

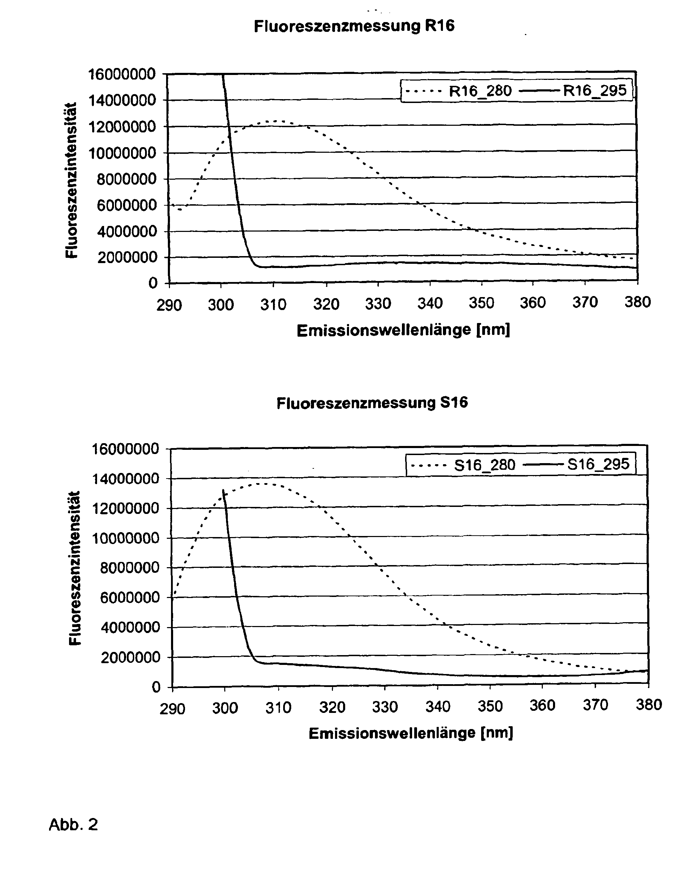

Die Aufreinigung des Proteins wurde nach folgendem Protokoll durchgeführt:
- Resuspension des Zellpellets in 5 ml pro Gramm Feuchtmasse 20 mM MOPS (3-(N-Morpholino)propanesulfonic acid) pH 7,0
- Aufschluß der Zellen im Hochdruckhomogenisator bei 1400 Bar
- Zentrifugation 30 min, 5000xg
- Waschen des Pellets mit 5 ml pro Gramm Feuchtmasse 50 mM Tris/HCl pH 8,0; 100 mM NaCl, 0,5 mM Ethylendiamintetraacetat (EDTA), 0,1% Triton X-100
- Waschen des Pellets mit 5 ml pro Gramm Feuchtmasse 50 mM Tris/HCl pH 8,0; 100 mM NaCl, 0,5 mM Ethylendiamintetraacetat (EDTA)
- Lösen des Pellets durch Zugabe von 1,6 g Guanidiniumthiocyanat (GdmSCN) pro Gramm Pellet (Endkonzentration 6 M GdmSCN)
- Zentrifugation 60 min, 5000xg
- Dialyse des Überstandes gegen 5 mM Kaliumphosphat pH 7,0
- Zentrifugation 30 min, 5000xg
- Fällung des Proteins aus dem Überstand durch Zugabe von ¼ Volumen 4 M Ammoniumsulfat 1 h bei Raumtemperatur
- Waschen des Pellets mit 8 M Harnstoff
- 2 x Waschen des Pellets mit Wasser
- Lyophilisierung
- Das lyophilisierte Protein kann bei -20°C gelagert werden.
- Für die Verwendung des Proteins wird das lyophilisierte Protein in 6 M GdmSCN aufgenommen, und gegen 5 mM Kaliumphosphat pH 7,0 dialysiert.
- Der Proteingehalt in Lösung wurde photometrisch bestimmt (E_{(280;1mg/ml)}=0,42)
- Die Reinheit des Proteins wurde durch SDS-PAGE und Westernblot Analyse (Abb.1), sowie durch Fluoreszenzspektroskopie überprüft (Abb.2)

Aus einer 10 l Fermentation wurden 20 g sauberes R₁₆-Protein mit einer Reinheit von >95% gewonnen.

### Beispiel 2

### Herstellung des repetitiven Proteins S₁₆ (SEQ ID NO:4)

Ein synthetisches Gen, das für das repetitives Protein S₁₆ kodiert, wurde durch Multimerisierung eines synthetischen Oligonukleotides S: entspreched der in Huemmerich et al.; Biochemistry 43(42):13604-12, (2004) beschriebenen Methode zu einem 16mer multimerisiert und in den Expressionsvektor pET21a (Novagen) kloniert. Die Expression erfolgte in dem Stamm E.coli BLR [DE3] (Novagen).
Die Anzucht und Proteinsynthese erfolgte bei pO₂>20% und pH=6,8 im Fed-Batch Verfahren.

### Medium

| 8 Liter | Wasser |
|---|---|
| 230 g | Hefe-Extrakt |
| 150 g | Bacto-Trypton |
| 30 g | Glycerin (99 %) |
| 20 g | Kaliumdihydrogenphosphat (KH₂PO₄) |
| 50 g | Ammoniumsulfat ((NH₄)₂SO₄) |
| 10,5 g | Magnesiumsulfatheptahydrat (MgSO₄*7 H₂O) |
| 1,0 g | Calciumchloriddihydrat (CaCl₂*2 H₂O) |
| 100 ml | Spurensalzlösung |
| | mit Wasser auf 9,7 Liter auffüllen pH-Wert mit 25%iger NaOH auf 6,8 einstellen |
| 3 ml | Tego KS 911 (Antifoam; Goldschmidt Produkte) |
| 1g | Ampicillin |

### Spurensalzlösung:

| 5 Liter | Wasser |
|---|---|
| 200,00 g | Citronensäuremonohydrat |
| 55,00 g | ZnSO₄*7H₂O |
| 42,50 g | (NH₄)₂Fe(SO₄)₂*6H₂O |
| 15,00 g | MnSO₄*H₂O |
| 4,00 g | CuSO₄*5H₂O |
| 1,25 g | CoSO₄*7 H₂O |

### Feeding Lösung

| | |
|---|---|
| 800 g | Wasser |
| 30 g | Citronensäure Monohydrat |
| 60 g | Natriumsulfat (Na₂SO₄) |
| 4,5 g | (NH₄)₂Fe(SO₄)₂*6H₂O |
| 3400 g | Glycerin 99,5 % |

Nach Aufbrauchen des im Grundmedium enthaltenen Glycerins wurde ein konstanter Feed bei einer Rate von 100 ml/h gestartet.

Die Proteinsynthese wurde durch Zugabe von 1 mM Isopropyl-β-D-thiogalactopyranosid induziert, nachdem die Bakterienkultur eine optische Dichte OD₆₀₀=40 erreichte. Zu diesem Zeitpunkt wurde die Temperatur der Kultur von 37°C auf 25°C gesenkt. 4 h nach Induktion wurden die Zellen geerntet.

Die Aufreinigung des Proteins wurde nach folgendem Protokoll durchgeführt:
- Resuspension des Zellpellets in 5 ml pro Gramm Feuchtmasse 20 mM MOPS (3-(N-Morpholino)propanesulfonic acid) pH 7,0
- Aufschluß der Zellen im Hochdruckhomogenisator bei 1400 bar
- Zentrifugation 30 min, 5000xg
- Inkubation des Überstandes 20 min bei 80°C
- Zentrifugation 30 min, 5000xg
- Fällung des Proteins aus dem Überstand durch Zugabe von 1 Volumen 4 M Ammoniumsulfat 1 h bei Raumtemperatur
- Waschen des Pellets mit 8 M Harnstoff
- 2 x Waschen des Pellets mit Wasser
- Lyophilisierung
- Das lyophilisierte Protein kann bei -20°C gelagert werden.
- Für die Verwendung des Proteins wird das Ammoniumsulfat-Präzipitat in 6 M GdmSCN aufgenommen, und gegen 5 mM Kaliumphosphat pH 7,0 dialysiert.
- Der Proteingehalt in Lösung wurde photometrisch bestimmt (E_{(280;1mg/ml)}=0,76)
- Die Reinheit des Proteins wurde durch SDS-PAGE und Westernblot Analyse (Abb.1), sowie durch Fluoreszenzspektroskopie überprüft (Abb.2)

Aus einer 10 l Fermentation wurden 1,4 g reines S₁₆-Protein gewonnen.

### Beispiel 3

### Bildung von Protein Microbeads aus repetitiven Proteinen R₁₆ und S₁₆

### Protokoll

- Lösen des lyophilisierten R₁₆ bzw. S₁₆ in 6 M Guanidiniumthiocyanat mit einer Endkonzentration von 10 mg/ml
- Dialyse gegen 1 M Kaliumphosphat pH 7,0 bei Raumtemperatur
- Waschen mit Wasser
- Lyophilisierung

Durch den Austausch des chaotropen Guanidiniumthiocyanates durch das kosmotrope Kaliumphosphat wird die Bildung von R₁₆- bzw. S₁₆-Microbeads induziert. Alternativ kann das Guanidiniumthiocyanat durch Dialyse gegen 5 mM Kaliumphosphat entfernt werden. Die Microbeadbildung wird durch Zugabe eines ¼ Volumens 4 M Ammoniumsulfat oder eines ½ Volumens 1 M Kaliumphosphat induziert.

### Protokoll

- Lösen des lyophilisierten R₁₆ bzw. S₁₆ in 6 M Guanidiniumthiocyanat mit einer Endkonzentration von 15 mg/ml
- Dialyse gegen 5 mM Kaliumphosphat pH 7,0 bei 4°C
- Entfernen von Prezipitat durch Zentrifugation für 30 min bei 10000xg
- Einstellung der Proteinkonzentration auf 10 mg/ml
- Zugabe von ¼ Volumen 4 M Ammoniumsulfat oder ½ Volumens 1 M Kaliumphosphat
- Inkubation bei Raumtemperatur für 1 h
- Waschen mit Wasser
- Lyophilisierung

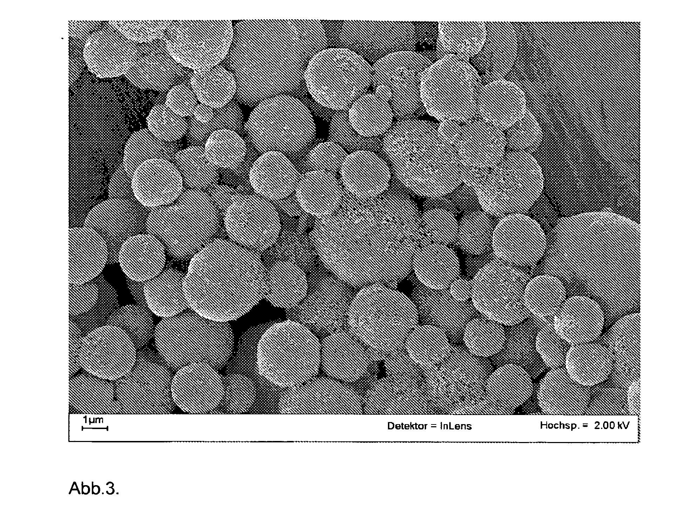

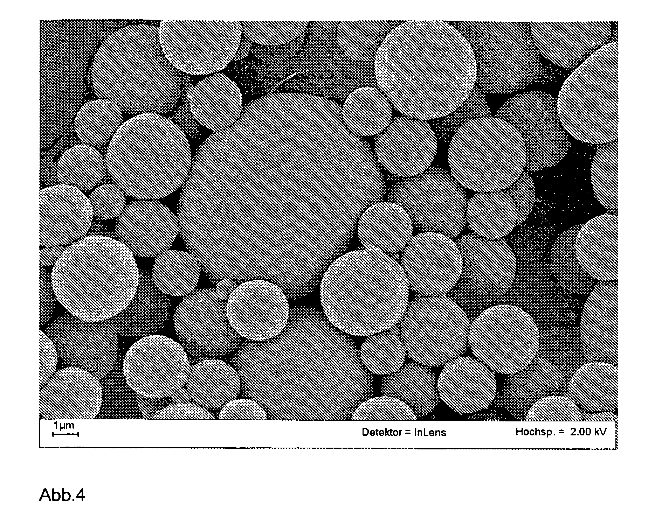

Der Erfolg der Microbead-Bildung wird mit Hilfe der Rasterelektronenmikroskopie Überprüft (Abb.3 + 4).

### Beispiel 4

### Stabilisierung von S₁₆ Microbeads

Der Bildungsprozess der S₁₆ Microbeads beinhaltet eine durch Kaliumphosphat oder Ammoniumsulfat induzierte Phasentrennung und die anschließende Stabilisierung der proteinreichen Phase durch Ausbildung stabiler Sekundärstrukturen. Die nicht ausgehärteten proteinreichen Tröpfchen können von stabilisierten Microbeads experimentell durch ihre unterschiedliche Stabilität in 4 M Harnstoff unterschieden werden. Die Kinetik der Stabilisierung wurde untersucht.

### Protokoll

- Präparation einer wässrigen 10 mg/ml S₁₆-Lösung in 5 mM Kaliumphosphat pH 7,0
- Fällung des S₁₆-Proteins zum Zeitpunkt 0 mit ¼ Volumen 4 M Ammoniumsulfat
- Entnahme eines Aliquots der gefällten Proteinsuspension zum Zeitpunkt t und Mischen mit dem gleichen Volumen 8 M Harnstoff
- Messung der Extinktion der Probe bei 600 nm als qualitatives Maß für die Streuung

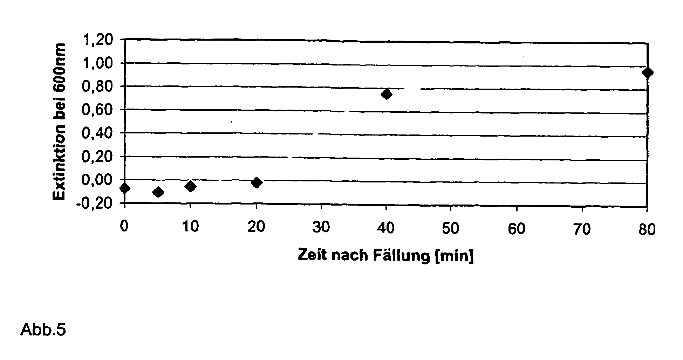

Innerhalb von 30 min kann die durch die Fällung verursachte Trübung der Probe durch Einstellen einer Konzentration von 4 M Harnstoff wieder rückgängig gemacht werden. Nach 40 min bleibt die Trübung bei dieser Behandlung erhalten. Es kann daher geschlossen werden, dass sich in diesem Zeitraum in der proteinreichen Phase eine Struktur ausgebildet hat, die stabil in 4 M Harnstoff ist (Abb.5).

### Beispiel 5

### Verpackung von β-Carotin in R₁₆- und S₁₆-Microbeads

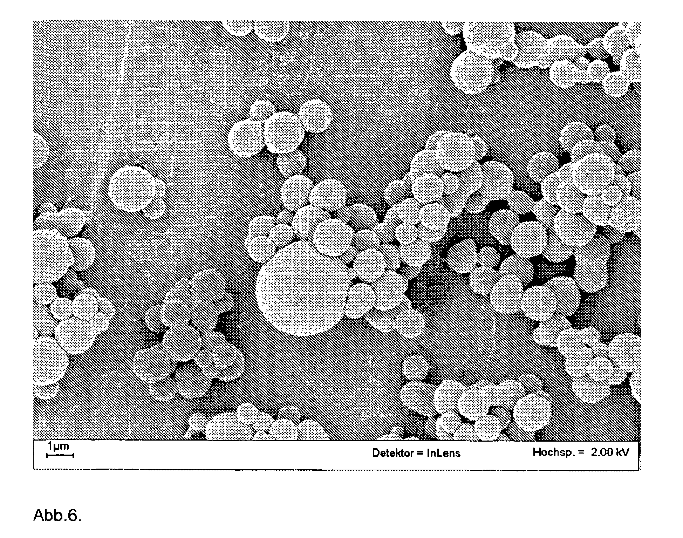

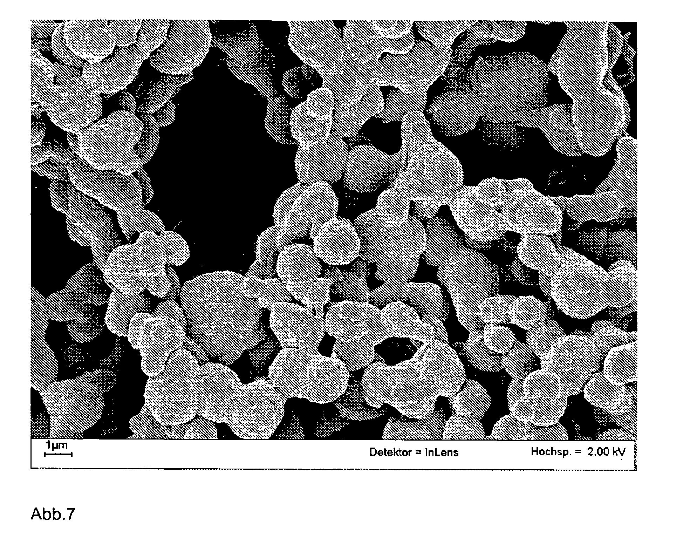

Um zu zeigen, dass die R₁₆- und S₁₆-Microbeads als Träger und Formulierungshilfsmittel für Wirkstoffe geeignet sind, wurde β-Carotin als Beispiel für schwer wasserlösliche Wirkstoffe in R₁₆- und S₁₆-Microbeads verpackt.
Dazu wurden 500 µl einer Lösung aus 10 mg/ml R₁₆ bzw. S₁₆ in 5 mM Kaliumphosphat (pH 8,0) mit 50 µl einer Lösung aus 0,9 mg/ml β-Carotin in 10% Tetrahydrofuran (THF) und 90% N-Methyl-2-pyrrolidon (NMP) vermischt. Im Anschluss wurde die Bildung von Microbeads durch Zugabe von 275 µl 4 M Ammoniumsulfatlösung induziert.
Während der Bildung der R₁₆- und S₁₆-Microbeads wurde das β-Carotin aus der Lösung entfernt. Die abzentrifugierten Microbeads erschienen gelb-orange gefärbt. Die Microbeads wurden mit Wasser gewaschen, wobei das β-Carotin mit den Microbeads assoziiert blieb und anschließend lyophilisiert. Nach dem Lyophilisieren wurden die beladenen R₁₆- bzw. S₁₆-Microbeads rasterelektronenmikroskopisch analysiert (Abb.6 + 7). Im elektronenmikroskopischen Bild zeigten die beladenen Microbeads (Abb.6 + 7) eine sehr ähnliche Morphologie wie die unbeladenen Microbeads (Abb.3 + 4), wodurch deutlich wird, dass die Microbeads und das β-Carotin eine gemeinsame feste Phase bilden.

### Beispiel 6

### Wasseraufnahme von R₁₆-Microbeads

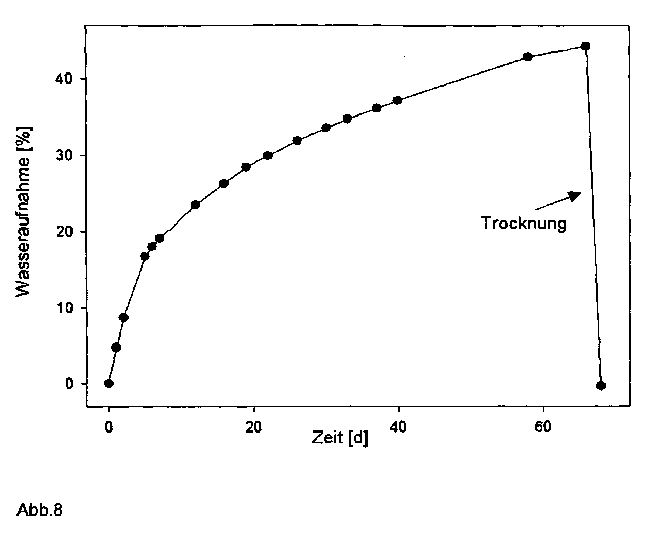

Microbeads aus repetitiven Proteinen können Wasser aus der Luft aufnehmen bzw. abgeben und damit als feuchtigkeitsregulierende Substanz z.B. in kosmetischen Applikationen dienen.
Um dies zu demonstrieren, wurden 0,4 g R₁₆-Microbeads, die zuvor bei -20°C gelagert wurden, 16 h unter Vakuum getrocknet und das Trockengewicht bestimmt. Anschließende Lagerung bei Raumtemperatur und 100 % Luftfeuchtigkeit führte zu einer langsamen Gewichtszunahme von 44% innerhalb von 66 Tagen (Abb.8). Durch erneute Trocknung im Vakuum wurde das ursprüngliche Trockengewicht wieder erreicht.

### Beispiel 7

### Gelbildung von R₁₆ und S₁₆

### Protokoll:

- Lösen des lyophilisierten R₁₆ bzw. S₁₆ in 6M Guanidiniumthiocyanat mit einer Endkonzentration von 20 mg/ml
- Dialyse gegen 5 mM Kaliumphosphat pH 7,0 bei 4°C
- Entfernen von Prezipitat durch Zentrifugation für 30 min bei 10000xg
- Herstellen einer Lösung mit 2,5 mM Kaliumphosphat pH 7,0, 10 mg/ml R₁₆-bzw. S₁₆-Protein und 40% v/v Ethanol
- Inkubation bei Raumtemperatur

Innerhalb von 4 - 5 Tagen nimmt die Lösung einen gelartigen, trüben Zustand an.

### Beispiel 8

### Filmildung von R₁₆ und S₁₆

### Protokoll

- Lösen des lyophilisierten R₁₆ bzw. S₁₆ in Hexafluorisopropanol mit einer Endkonzentration von 100 mg/ml
- Ausplattieren von 300 µl auf einer glatten Polystyroloberfläche
- Abdampfen des Hexafluorisopropanols bei Raumtemperatur
- Überschichtung des Proteinfilms für 5 min mit 1 M Kaliumphosphat pH 7.0
- 2 x Waschen mit Wasser
- Lufttrocknung

Durch diese Behandlung entstehen transparente, wasserunlösliche Proteinfilme. Trockene Filme aus R₁₆ sind spröde, wogegen Filme aus S₁₆ flexible Eigenschaften aufweisen.

### Verzeichnis der Abbildungen

Abb.1. Analyse der gereinigten Proteine R₁₆ und S₁₆. Eine wässrige Lösung der Proteine wurde hergestellt und elektrophoretisch auf einem Polyacrylamidgel aufgetrennt. Die Detektion der Proteine erfolgte über eine Färbung mit dem Farbstoff Coomassie Brilliant Blau (C) bzw. über Immunoblotting mit einem Antikörper, der spezifisch an den T7-Peptidtag bindet, der beiden Protein aminoterminal angefügt ist (WB).

Abb. 2 Reinheitsanalyse des gereinigten R₁₆ und S₁₆ mittels Fluoreszenzspektroskopie. R₁₆ und S₁₆ enthalten nicht die Aminosäure Tryptophan, während Tyrosine vorhanden sind. Dagegen ist Tryptophan mit einer durchschnittlichen Häufigkeit von 1,5% in Proteinen von E.coli vorhanden. Durch Fluoreszenzspektroskopie kann die Anwesenheit von Tryptophanen in einer Proteinprobe und damit von Verunreinigungen in R₁₆-bzw. S₁₆-Präparationen selektiv nachgewiesen werden. Während durch Licht der Wellenlänge 280 nm (blau) sowohl Tyrosine als auch Tryptophane angeregt werden, führt Licht mit 295 nm (magenta) ausschließlich zur Anregung von Tryptophan. Die R₁₆- und S₁₆-Proben zeigen eine für Tyrosin typische Fluoreszenz, während kein Anzeichen für die Anwesenheit von Tryptophan detektierbar ist, was ein Hinweis auf die hohe Reinheit der Proteinproben ist.

Abb.3 R₁₆-Microbeads. Die Microbeads wurden durch Dialyse einer 10 mg/ml R₁₆-Lösung in 6 M GdmSCN gegen eine 1 M Kaliumphosphatlösung pH 7,0 erhalten und mittels Rasterelektronenmikroskopie analysiert.

Abb.4 S₁₆-Microbeads. Die Microbeads wurden durch Dialyse einer 10 mg/ml S₁₆-Lösung in 6 M GdmSCN gegen eine 1 M Kaliumphosphatlösung pH 7,0 erhalten und mittels Rasterelektronenmikroskopie analysiert.

Abb.5 Stabilisierung von S₁₆-Microbeads. Eine wässrige 10 mg/ml Sie-Lösung wurde durch Zugabe von ¼ Volumen Ammoniumsulfat zum Zeitpunkt t = 0 gefällt. Nach definierten Zeitpunkten wurden Aliquots entnommen, eine Konzentration von 4 M Harnstoff eingestellt, und die Extinktion bei 600 nm als qualitatives Maß für die Streuung und damit für die Anwesenheit stabiler Proteinpartikel gemessen.

Abb.6 R₁₆-Microbeads mit 0,8% β-Carotin. Die Microbeads wurden durch Zugabe einer 4 M Ammoniumsulfatlösung zu 10 mg/ml R₁₆ und 0,08 mg/ml β-Carotin in 6 M GdmSCN erhalten und mittels Rasterelektronenmikroskopie analysiert

Abb.7 S₁₆-Microbeads mit 0,8% β-Carotin. Die Microbeads wurden durch Zugabe einer 4 M Ammoniumsulfatlösung zu 10 mg/ml S₁₆ und 0,08 mg/ml β-Carotin in 6 M GdmSCN erhalten und mittels Rasterelektronenmikroskopie analysiert.

Abb.8 Feuchtigkeitsaufnahme von R₁₆-Microbeads. Die Aufnahme von Wasser wurde durch die Gewichtszunahme von R₁₆-Microbeads bei 100% Luftfeuchtigkeit ermittelt

## Patentansprüche

1. Repetitives Protein mit Wiederholungseinheiten, das die Wiederholungseinheit PGSSAAAAAAAASGPGQGQGQGQGQGGRPSDTYG enthält.

2. Repetitives Protein mit Wiederholungseinheiten, das die Wiederholungseinheit SAAAAAAAAGPGGGNGGRPSDTYGAPGGGNGGRPSSSYG enthält.

3. Repetitives Protein nach Anspruch 1, **dadurch gekennzeichnet, dass** es die Aminosäuresequenz SEQ ID NO:2 enthält.

4. Repetitives Protein nach Anspruch 2, **dadurch gekennzeichnet, dass** es die Aminosäuresequenz SEQ ID NO:4 enthält.

5. Eine Nukleinsäuresequenz, die für ein repetitives Protein gemäß einem der Ansprüche 1-4 kodiert.

6. Ein Wirtsorganismus, der eine Nukleinsäuresequenz des Anspruchs 5 enthält.

7. Verfahren zur Herstellung eines repetitiven Proteins gemäß einem der Ansprüche 1-4, **dadurch gekennzeichnet, daß** das Protein von einem. Wirtsorganismus ensprechend des Anspruchs 6 hergestellt wird und anschließend isoliert wird.

8. Die Verwendung eines repetitiven Proteins gemäß einem der Ansprüche 1-4 in der Kosmetik.

9. Die Verwendung eines repetitiven Proteins, gemäß einem der Ansprüche 1-4 für die Formulierung von Wirk- und Effektstoffen.

10. Die Verwendung eines repetitiven Proteins gemäß einem der Ansprüche 1-4 für die Papier-, Leder- und Textilveredelung.

11. Die Verwendung eines repetitiven Proteins gemäß einem der Ansprüche 1-4 zum Beschichten von Oberflächen.

12. Die Verwendung eines repetitiven Proteins gemäß einem der Ansprüche 1-4 in der Human- und Tierernährung.

## Claims

1. A repetitive protein having repetition units, comprising the repetition unit PGSSAAAAAAAASGPGQGQGQGQGQGGRPSDTYG.

2. A repetitive protein having repetition units, comprising the repetition unit SAAAAAAAAGPGGGNGGRPSDTYGAPGGGNGGRPSSSYG.

3. The repetitive protein according to claim 1, wherein said protein comprises the amino acid sequence SEQ ID NO: 2.

4. The repetitive protein according to claim 2, wherein said protein comprises the amino acid sequence SEQ ID NO: 4.

5. A nucleic acid sequence coding for a repetitive protein according to any of claims 1-4.

6. A host organism comprising a nucleic acid sequence of claim 5.

7. A process for producing a repetitive protein according to any of claims 1-4, wherein the protein is produced by a host organism according to claim 6 and subsequently isolated.

8. The use of a repetitive protein according to any of claims 1-4 in cosmetics.

9. The use of a repetitive protein according to any of claims 1-4 for the formulation of active compounds and effectors.

10. The use of a repetitive protein according to any of claims 1-4 for refining paper, leather and textiles.

11. The use of a repetitive protein according to any of claims 1-4 for the coating of surfaces.

12. The use of a repetitive protein according to any of claims 1-4 in human and animal nutrition.

## Revendications

1. Protéine répétitive ayant des unités récurrentes, qui contient l'unité récurrente **PGSSAAAAAAAASGPGQGQGQGQGQGGRPSDTYG**

2. Protéine répétitive ayant des unités récurrentes, qui contient l'unité récurrente **SAAAAAAAAGPGGGNGGRPSDTYGAPGGGNGGRPSSSYG**

3. Protéine répétitive selon la revendication 1, **caractérisée en ce qu'**elle contient la séquence d'acides aminés SEQ ID NO:2.

4. Protéine répétitive selon la revendication 2, **caractérisée en ce qu'**elle contient la séquence d'acides aminés SEQ ID NO:4.

5. Séquence d'acide nucléique, qui code pour une protéine répétitive selon l'une des revendications 1-4.

6. Organisme hôte, qui contient une séquence d'acide nucléique selon la revendication 5.

7. Procédé de fabrication d'une protéine répétitive selon l'une des revendications 1-4, **caractérisé en ce que** la protéine est fabriquée à partir d'un organisme hôte correspondant à la revendication 6, puis est isolée.

8. Utilisation d'une protéine répétitive selon l'une des revendications 1-4 en cosmétique.

9. Utilisation d'une protéine répétitive selon l'une des revendications 1-4 pour la formulation de matières actives ou de substances conférant un effet.

10. Utilisation d'une protéine répétitive selon l'une des revendications 1-4 pour le finissage du papier, du cuir et des textiles.

11. Utilisation d'une protéine répétitive selon l'une des revendications 1-4 pour revêtir des surfaces.

12. Utilisation d'une protéine répétitive selon l'une des revendications 1-4 en alimentation humaine et animale.
